(19) Europäisches Patentamt / European Patent Office / Office européen des brevets

(11)  **EP 4 393 951 A1**

(12) **EUROPEAN PATENT APPLICATION**
published in accordance with Art. 153(4) EPC

(43) Date of publication:
03.07.2024 Bulletin 2024/27

(21) Application number: 22860589.5

(22) Date of filing: 25.08.2022

(51) International Patent Classification (IPC):
C07K 16/28 (2006.01)        C12N 15/13 (2006.01)
A61K 39/395 (2006.01)       A61K 47/68 (2017.01)
A61K 45/06 (2006.01)        A61K 31/704 (2006.01)
A61P 35/00 (2006.01)

(52) Cooperative Patent Classification (CPC):
A61K 31/704; A61K 39/395; A61K 45/06;
A61K 47/68; A61P 35/00; C07K 16/00; C07K 16/28

(86) International application number:
PCT/CN2022/114793

(87) International publication number:
WO 2023/025243 (02.03.2023 Gazette 2023/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.08.2021 CN 202110999986

(71) Applicant: CSPC Megalith Biopharmaceutical Co.,
Ltd.
Shijiazhuang, Hebei 050025 (CN)

(72) Inventors:
• SUN, Zhaopeng
  Shijiazhuang, Hebei 050025 (CN)
• SHEN, Mingyue
  Shijiazhuang, Hebei 050025 (CN)
• DAN, Mo
  Shijiazhuang, Hebei 050025 (CN)
• YUAN, Can
  Shijiazhuang, Hebei 050025 (CN)

• WANG, Yancui
  Shijiazhuang, Hebei 050025 (CN)
• WU, Yufen
  Shijiazhuang, Hebei 050025 (CN)
• HUI, Xiwu
  Shijiazhuang, Hebei 050025 (CN)
• LIU, Boning
  Shijiazhuang, Hebei 050025 (CN)
• YAO, Bing
  Shijiazhuang, Hebei 050025 (CN)

(74) Representative: Longland, Emma Louise et al
Venner Shipley LLP
Byron House
Cambridge Business Park
Cowley Road
Cambridge CB4 0WZ (GB)

Remarks:
The complete document including Reference
Table(s) and the Sequence Listing(s) can be
downloaded from the EPO website

(54) **ANTI-NECTIN-4 ANTIBODY, DRUG CONJUGATE, AND PREPARATION METHOD THEREFOR AND USE THEREOF**

(57)  The present application relates to an anti-Nectin-4 antibody, a drug conjugate and a preparation method and use thereof, which can be effectively used for the treatment and/or prevention of Nectin-4 positive tumors, including cancers, such as breast cancer and bladder cancer.

**Description**

**FIELD**

The present invention relates to the field of biomedicine, in particular to an anti-Nectin-4 protein antibody and a conjugate thereof, as well as the use of the antibody and the conjugate in the treatment of tumors.

**BACKGROUND**

Nectin-4 (Nectin Cell Adhesion Molecule 4), also known as PVRL4 (Poliovirus Receptor like 4), is a type I membrane protein that is a member of the cell adhesion factor ( Nectin ) family, which is specifically expressed in the embryo and placenta; this family is a group of $Ca^{2+}$ independent immunoglobulin-like transmembrane cell adhesion molecules consisting of four members (Nectin-1 to Nectin-4). Nectin-1 to Nectin-3 are widely expressed in normal adult tissues. Nectin-4 protein cannot be detected in healthy adult tissues (including breast tissues), but is overexpressed in a variety of cancer (breast, lung, ovarian, etc.) tissues and is found to be overexpressed in 62% of triple-negative breast cancers.

Studies have shown that overexpression of Nectin-4 in cancer progression can promote intratumoral angiogenesis and promote tumor growth. In addition, the PI3K/AKT signaling pathway is involved in the promotion of Nectin-4-mediated cancer cell proliferation. Furthermore, overexpression of Nectin-4 is associated with poor prognosis of lung, breast and ovarian cancers. Nectin-4 interacts with Afadin protein ( actin filament-binding protein) to form a complex, and ultimately improves cell survival and prevents apoptosis through PI3K-AKT signaling pathway; high expression of Nectin-4 is a risk factor for lymph node metastasis in papillary thyroid carcinoma (PTC) patients; Nectin-4 depletion can effectively inhibit the proliferation and invasion of two PTC cell lines (ie, TPC1 and KTC-1 human thyroid cancer cell lines) and induce apoptosis in vitro; Overexpression of Nectin-4 in human esophageal cancer tissue is closely related to the size of the tumor, the depth of tumor invasion and the poor prognosis of the patients. Intervention of Nectin-4 expression in esophageal cancer cell lines show that increased Nectin-4 expression can significantly promote cell viability, migration, invasion and tumor formation.

Bladder cancer is a malignant tumor that occurs on the bladder mucosa, most of which are transitional cell carcinomas. It is most common in the lateral and posterior walls of the bladder, followed by the trigone and apex, which can occur polycentrically. Bladder cancer is the most common tumor of the urinary system and is more common in men. According to the data released by the National Cancer Center in 2019, bladder cancer ranks seventh among male cancers, with an incidence rate of 8.83/100,000, and the rising trend is more obvious in recent years. There are differences in the incidence of bladder cancer by region, race and gender, and it can occur in all age groups. High incidence occurs in the age between 50 and 70 years old, and the incidence rate gradually increases with age.

Bladder cancers are clinically classified as non-muscle invasive bladder cancer (NMIBC) and muscle invasive bladder cancer (MIBC) or metastatic tumors. Urothelial (transitional) carcinoma is a common type of bladder cancer, accounting for about 90% of all cases. Metastatic bladder cancer is generally associated with a poor prognosis, with a 5-year survival rate of only 15% for patients with stage IV bladder cancer. For NMIBC and MIBC, transurethral resection of bladder tumor (TURBT) is the standard therapy, with local intravesical chemotherapy followed by immunotherapy (with Bacillus Calmette-Guerin (BCG)) as adjuvant therapy; Chemotherapy has already become the first choice for cancer treatment during the past few decades The mostly commonly used is platinum-based therapy.

Breast cancer is still the most common female tumor in the world. The incidence of breast cancer in the world increases by 0.2% to 8% each year. About 1.4 million people are diagnosed with breast cancer, wherein about 0.5 million people die from the disease each year. Breast cancer is the leading cause of death in women aged 40-55. Since the late 1970s, the incidence of breast cancer has been ranked first among female malignant tumors worldwide. According to figures released by the American Cancer Society, there are about 200,000 new breast cancer cases in the United States each year, with an incidence rate of 116/100,000. Although China is a low-incidence area of female breast cancer, the incidence of breast cancer has increased significantly in recent years. The incidence of breast cancer in major Chinese cities has increased by 37% in the past 10 years, and the mortality rate has increased by 38.9%. In particular, Shanghai, Beijing, Tianjin and coastal areas are the high incidence areas of breast cancer in China, which already account for the first place in the incidence of female malignant tumors.

At present, anti-tumor drugs in the world are mainly targeting drug, monoclonal antibodies, and monoclonal antibodies are the largest sub-industry in the field of biopharmaceuticals. Data show that in 2016, monoclonal antibody drugs accounted for 43% of the total biological drug market share. Although monoclonal antibody therapy has the characteristics of high target specificity and low side effects, its efficacy is relatively limited when used alone. Therefore, most monoclonal antibody drugs are used in combination with chemotherapy, and the main way to improve the efficacy of monoclonal antibodies is antibody drug conjugates. Antibody-drug conjugates belong to a new class of anti-cancer bio-missile drugs, which are composed of three parts: an antibody, a drug and a linker connecting the two. After the monoclonal antibody is coupled with the drug, the antibody-drug conjugate uses the targeting property of the monoclonal antibody to specifically recognize the receptor on the surface of the cancer cell, bind to the receptor, and then enter into the cell, and prevent the cancer cells from proliferating and kill the cancer cells by using proteases inside the cells to release the drug. Antibody-drug conjugation technology integrates small molecule drugs with biological proteins, and has the advantages of both, which greatly enhances drug efficacy and reduces toxic and side effects, making it a new generation of therapeutic products.

At present, there is no chemical drug, but only 6 biological drugs in the world which targets Nectin-4. Among them, there is only one approved antibody drug conjugate (ADC) targeting Nectin-4, which is an ADC drug targeting Nectin-4 jointly developed by Agensys company, Japan (a subsidiary of Astellas) and Seattle Genetics company, USA. It was accelerated approved by the FDA on December 19, 2019. The trade name is Padcev, and the indications are: locally advanced or metastatic urothelial carcinoma. Relevant clinical data showed that: ORR was 44%, CR was 12%, and median DOR was 7.6 months; 46% of patients treated with Padcev experienced serious adverse reactions; the most common serious adverse reactions ($\geq$3%) were urinary tract infection (6%), cellulitis (5%), febrile neutropenia (4%), diarrhea (4%), sepsis (3%), acute kidney injury (3%), dyspnea (3%), and rash (3%); fatal adverse reactions occurred in 3.2% of patients, including acute respiratory failure (0.8%), aspiration pneumonitis (0.8%), cardiac diseases (0.8%) and sepsis (0.8%).

It can be seen that the adverse reactions of Padcev are relatively serious. Based on the research status of Nectin-4 target, patients still have unmet therapeutic needs for related antibody-drug conjugates, and there is an urgent need for efficient, low-side effects, and affordable anti-tumor targeting drugs that can benefit the majority of tumor patients.

The development of ADCs is a very complex task. Correct selection of targets, optimization and improvement of drugs, and selection of appropriate linkers to improve the efficacy and safety of ADC drugs are all difficult points in ADC drug development. ADC drug design needs to be based on comprehensive consideration of antibodies, linkers and drugs as an organic whole, to clarify tumor indications and drug targets, and to fully and thoroughly study the mechanism of action of ADC drugs, so as to finally realize the effective killing of ADC drugs on tumor cells, while improving the quality of life of patients.

It should be emphasized that one of the keys to the success of ADCs lies in the selection of appropriate antibodies to deliver drugs to tumor cells under the condition that the drug activity is maintained. Antibodies used to prepare ADCs must have certain characteristics. Not only do they need to specifically bind to antigen-positive cells in tumors, but also the antigen-antibody complexes need to be able to mediate the internalization of ADCs. At least the following three factors should be considered when screening antibodies: specific binding, internalization, and cellular localization of the antibody after internalization; and, there is no clear link between the affinity of the antibody and the rate of internalization (Laurent Ducry, Antibody Drug Conjugates, Science Press, pp. 36-37 Page). Therefore, as a highly accurate "localization device", antibodies can specifically bind to target cells and effectively mediate "endocytosis" and their localization, which are crucial to the specificity and toxicity of antibody-drug conjugates.

Based on the defects of the prior art, the inventors aim at the problem of unstable conjugation between the linker of the existing drug and the small-molecule drugs, and adopt $NH_2$-$PEG_3$-Val-Cit as the linker. The humanized antibody is coupled to a small molecule drug (for example: MMAE ) through a linker by enzyme mediated site-directed coupling, and the obtained ADC drug has a strong killing effect on tumor cells with high Nectin-4 expression, especially breast cancer, bladder cancer and other tumors. Specifically, first of all, according to the results of the endocytosis experiment, the anti-Nectin-4 antibody drug conjugate obtained by the present invention has better endocytosis effect than the control drug Padcev, and can simultaneously achieve specific binding, efficient internalization and precise cell localization. The specificity of the antibody-drug conjugate is increased while its toxicity was reduced. Secondly, in vivo experiments show that intravenous administration of the antibody-drug conjugate in nude mice with Nectin-4- positive xenograft tumors result in tumor growth inhibition, and a significant therapeutic effect can be observed under a single dose intravenous administration of as low as 1 mg/kg; its overall therapeutic effect is significantly better than that of the control drug Padcev. Finally, the second administration of Padcev at 6 mg/kg in the monkey toxicology experiment lead to serious adverse reactions and fatality. In contrast, the antibody drug conjugate obtained by the present invention has basically no adverse reactions in two administrations at a high dose of 9 mg/kg, has a wider therapeutic window period, and has better druggability. In summary, the antibody drug conjugates provided by the present application have achieved unexpected technical results.

## SUMMARY OF THE INVENTION

The present invention provides an antibody, and a functional fragment (eg, an antigen-binding fragment) thereof, and an antibody drug conjugate (ADC) that bind to Nectin-4 protein and/or Nectin-4 protein or polypeptide fragment.

In one aspect, the invention provides an antibody or functional fragment thereof capable of specifically binding Nectin-4, the antibody comprising heavy chain(s) and light chain(s), wherein

(i) the heavy chain comprises three CDR regions, wherein the amino acid sequence of at least one of the CDR regions has the amino acid sequence shown in SEQ ID NO : 1, 2 or 3 or a sequence having at least 80 % ( preferably 85 %, 90 %, 95 %, 96 %, 97 %, 98 % or 99 %) sequence identity thereto; and /or

(ii) the light chain comprises three CDR regions, wherein the amino acid sequence of at least one of the CDR regions has the amino acid sequence shown in SEQ ID NO : 4, 5 or 6 or a sequence having at least 80 % ( preferably 85 %, 90 %, 95 %, 96 %, 97 %, 98 % or 99 %) sequence identity thereto.

In some embodiments, the antibody comprises heavy chain(s) and light chain(s), wherein

(i) the heavy chain comprises three CDR regions which are CDR1 region, CDR2 region and CDR3 region respectively, wherein said CDR1 region, CDR2 region and CDR3 region have the amino acid sequences shown in SEQ ID NOs : 1, 2 and 3, respectively; and/or

(ii) the light chain comprises three CDR regions which are CDR1 region, CDR2 region and CDR3 region respectively, wherein said CDR1 region, CDR2 region and CDR3 region have the amino acid sequences shown in SEQ ID NOs : 4, 5 and 6, respectively.

In some embodiments, the heavy chain variable region sequence of the antibody is an amino acid sequence shown in SEQ ID NO.9 or a sequence having at least 80% (preferably 85%, 90%, 95%, 96%, 97%, 98% or 99%) sequence identity thereto, and the light chain variable region sequence is selected from amino acid sequences shown in SEQ ID NOs.10-12 or a sequence having at least 80% (preferably 85%, 90%, 95%, 96%, 97%, 98% or 99%) sequence identity thereto.

In some embodiments, the antibody or functional fragment thereof of the invention is isolated.

In some embodiments, the antibody of the invention is a monoclonal antibody.

In some embodiments, the antibody or functional fragment thereof of the invention is a humanized antibody, preferably a fully human antibody.

In some embodiments, the antibody of the invention is a bispecific antibody.

In some embodiments, the antibody or functional fragment thereof of the invention has ADCC activity.

In some embodiments, the antibody or functional fragment thereof of the invention has CDC activity.

In some embodiments, the antibody or functional fragment thereof of the invention specifically binds Nectin-4 and substantially does not bind Nectin-1, Nectin-2, or Nectin-3.

In some embodiments, the antibody or functional fragment thereof of the invention includes: a diabody, an Fab fragment, an Fab ' fragment, an F(ab)'₂, an scFv, a dsFv and a single domain antibody. The scFv protein is a fusion protein which the light chain variable region and the heavy chain variable region of the immunoglobulin are linked through a linker; in the dsFv, cysteine is introduced at a specific site in the conserved framework regions of VH and VL, thereby introducing disulfide bond stabilized dsFv structure.

In some embodiments, the antibody or functional fragment thereof of the invention is IgM, IgD, IgG, IgA or IgE, wherein the IgG antibody has 4 subtypes: IgG1, IgG2, IgG3, IgG4, preferably IgG1 antibody.

In some embodiments, the constant region sequence of the heavy chain of the antibody is:

ASTKGPSVFPLAPSSKSTSGGTAALGCLVKDYFPEPVTVSWNSGALTSGVHTFPA

VLQSSGLYSLSSVVTVPSSSLGTQTYICNVNHKPSNTKVDKKVEPKSCDKTHTCPPCPA

PELLGGPSVFLFPPKPKDTLMISRTPEVTCVVVDVSHEDPEVKFNWYVDGVEVHNAKT

KPREEQYNSTYRVVSVLTVLHQDWLNGKEYKCKVSNKALPAPIEKTISKAKGQPREPQ

VYTLPPSREEMTKNQVSLTCLVKGFYPSDIAVEWESNGQPENNYKTTPPVLDSDGSFFL

YSKLTVDKSRWQQGNVFSCSVMHEALHNHYTQKSLSLSPGK (SEQ ID NO.16).

In some embodiments, the constant region sequence of the light chain of the antibody is:

RTVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO.17).

In some embodiments, the antibody or functional fragment thereof of the invention can be used to treat or prevent cancer, wherein the cancer overexpresses Nectin-4.

In some embodiments, the antibody or functional fragment thereof that has the ability to bind to Nectin-4 binds to a native epitope of Nectin-4 present on the surface of living cells. In some embodiments, the antibody or functional fragment thereof that has the ability to bind to Nectin-4 binds to the extracellular domain of Nectin-4. In some embodiments, the antibody or functional fragment thereof that has the ability to bind to Nectin-4 binds to the first extracellular domain of Nectin-4. In some embodiments, the antibody or functional fragment thereof that has the ability to bind to Nectin-4 binds to the 1-147aa domain of Nectin-4. In some embodiments, the antibody or functional fragment thereof that has the ability to bind to Nectin- 4 binds to the 32-142aa domain of Nectin-4.

In another aspect, the present invention provides an isolated polynucleotides encoding antibody of the present invention.

In yet another aspect, the invention provides a combination of isolated polynucleotides comprising a polynucleotide encoding the light chain of the antibody or functional fragment thereof of the invention and a polynucleotide encoding the heavy chain of the antibody or a functional fragment thereof of the invention.

In another aspect, the present invention provides an expression vector comprising the polynucleotide of the present invention or the combination of the polynucleotides of the present invention, wherein the polynucleotide is operably linked to a regulatory sequence which allows expression of a polypeptide encoded thereby in a host cell or in a cell-free expression system.

In some embodiments of the invention, the host cell may be a prokaryotic host cell, a eukaryotic host cell, or a bacteriophage. The prokaryotic host cell can be *Escherichia coli, Bacillus subtilis, Streptomyces* or *Proteus mirabilis* and the like. The eukaryotic host cell can be fungi such as *Pichia pastoris, Saccharomyces cerevisiae, Schizosaccharomyces,* and *Trichoderma,* an insect cell such as grass armyworm; a plant cell such as tobacco, mammalian cells, *i.e.* BHK cell, CHO cell, COS cell, myeloma cell and the like. In some embodiment, the host cell of the present invention is preferably a mammalian cell, more preferably BHK cell, CHO cell, NSO cell or COS cell.

In another aspect, the present invention provides an antibody drug conjugate comprising the antibody or functional fragment thereof of the present invention conjugated with one or more drugs, preferably the drugs are cytotoxic drugs (such as antimetabolites, antitumor antibiotics, alkaloids), immune enhancers or radioisotopes. More preferably, the drug is selected from auristatin derivatives, maytansinoid derivatives (such as Ansamitocin or Mertansine, dolastatin and derivatives thereof), camptothecin analogues, DNA topoisomerase I inhibitors and derivatives thereof. Most preferably, the drug is selected from MMAE (Monomethyl auristatin E) and MMAF (Monomethyl auristatin F).

In some embodiments, the antibody or functional fragment thereof having the ability to bind to Nectin-4 is covalently linked to the drug moiety through a linker. In some embodiments, the linker is a cleavable linker. In some embodiments, the linker is cleavable under intracellular conditions. In one embodiment, the linker is hydrolyzable at a pH of less than 5.5. In some embodiments, the linker is cleavable by intracellular proteases. In one embodiment, the linker is a cathepsin-cleavable linker. In some embodiments, the linker comprises a dipeptide, or a tetrapeptide. In some embodiments, the dipeptide is valine (Val)-citrulline (Cit). In some embodiments, the antibody is linked to a linker through a cysteine sulfhydryl group of the antibody. In one embodiment, the antibody is linked to a linker through an amino group of the antibody, particularly an amino group of a glutamine residue.

In some embodiments, the antibody drug conjugate of the present invention has the following general formula: Ab-(L-U)n, wherein Ab represents a monoclonal antibody targeting Nectin-4, L is a linker selected from $NH_2$-(PEG)m-Val-Cit, $NH_2$-(PEG)m-Val-Cit-pABC, mc-Val-Cit-pABC, Val-Cit-pABC, Val-Cit-pAB or Val-Cit, U is a drug selected from DM1, DM4, MMAE, MMAF, DXD and SN38; and m represents the number of PEG, which is an integer from 1 to 8, preferably 2, 3, 4, 5, 6, 7, or 8, more preferably it is 3, 4, 5, or 6, most preferably 3; n represents the drug-to-antibody ratio DAR, which is an integer from 1 to 8, preferably 2, 4, 6, or 8, more preferably 2, or 4, most preferably 2. In some specific embodiments, n is a decimal from 1 to 8. The full name of mc-Val-Cit-pABC is: maleimidocaproyl-valine-citrulline-p-aminocarbamate ; and the full name of Val-Cit-pAB is : valine-citrulline-p-aminobenzyloxycarbonyl.

In some embodiments, the present invention relates to an antibody drug conjugate (ADC) capable of specifically binding to Nectin-4, wherein the antibody or a functional fragment thereof comprises a heavy chain variable region and a light chain variable region, wherein the heavy chain variable region sequence is the amino acid sequence shown in SEQ ID NO.9; and the light chain variable region sequence is selected from the amino acid sequences shown in SEQ ID NO.10-12.

In some embodiments, the heavy chain variable region sequence of the antibody or the functional fragment thereof is an amino acid sequence shown in SEQ ID NO.9 or a sequence having at least 80%, 85%, 90%, 95%, 96%, 97%, 98% or 99% sequence identity thereto, and the light chain variable region sequence is selected from amino acid sequences shown in SEQ ID NOs. 10-12 or a sequence having at least 80%, 85%, 90%, 95%, 96%, 97%, 98% or 99% sequence identity thereto.

In some embodiments, the antibody is a monoclonal antibody.

In some embodiments, the antibody is a humanized antibody.

In some embodiments, the present invention relates to an isolated polynucleotide encoding a light chain of the antibody or functional fragment thereof and/or a heavy chain of the antibody or functional fragment thereof; or encoding the antibody or a functional fragment thereof.

In some embodiments, the present invention relates to an expression vector comprising the polynucleotide operably linked to a regulatory sequence which allows expression of a polypeptide encoded thereby in a host cell or in a cell-free expression system.

In some embodiments, the present invention relates to a host cell comprising the expression vector.

In another aspect, the present invention provides a pharmaceutical formulation comprising the antibody drug conjugate of the present invention, and a pharmaceutically acceptable diluent, carrier or excipient.

In another aspect, the present invention provides a medical preparation comprising the antibody drug conjugate of the present invention. In some embodiments, the medical preparation is in the form of a kit comprising a container containing the antibody drug conjugate. In some embodiments, the medical preparation further comprises printed instructions for use of the preparation in a method of treating or preventing a cancer, particularly a cancer expressing Nectin-4.

In another aspect, the present invention provides an antibody drug conjugate for effectively treating and/or preventing a tumor associated with a cell expressing Nectin-4.

In another aspect, the present invention provides use of the aforementioned antibody drug conjugate and anti-proliferative agent in the preparation of a medicament for treating a tumor.

In another aspect, the present invention provides a pharmaceutical composition comprising the aforementioned antibody drug conjugate and an anti-proliferative agent.

In certain embodiments, the anti-proliferative agent can also be an antibody, an antibody drug conjugate, or a fusion protein.

In another aspect, the present invention also relates to a method for treating a tumor in a subject, comprising administering the antibody drug conjugate or the pharmaceutical composition or the medical preparation to the subject.

In some embodiments, the present invention also relates to a method of treating a tumor in a subject, comprising administering to the subject an effective amount of the antibody drug conjugate or the pharmaceutical composition or the medical preparation and radiation.

In some embodiments, the present invention also relates to a method of treating a tumor in a subject, comprising administering to the subject an effective amount of the antibody drug conjugate or the pharmaceutical composition or the medical preparation and an anti-proliferative agent.

In another aspect, the present invention also relates to the aforementioned antibody, antibody drug conjugate and pharmaceutical composition for use in treating a tumor, preferably Nectin-4 positive tumor.

In some embodiments, tumors (including cancers) as described above include, but are not limited to, hematological tumors or solid tumors.

In some embodiments, tumors (including cancers) described above are hematological tumors, preferably lymphoma or leukemia, including but not limited to, myeloma, B-cell lymphoma, mantle cell lymphoma, non-Hodgkin B- cell lymphoma, non-Hodgkin lymphoma T-cell lymphoma, cutaneous lymphoma, anaplastic large cell lymphoma, multiple myeloma, indolent non-Hodgkin lymphoma, plasmacytoma, chronic lymphocytic leukemia, small lymphocytic lymphoma, and follicular lymphoma. In some embodiments, the hematological tumors are relapsed or refractory.

In some embodiments, tumors (including cancers) described above are solid tumors, including, but not limited to, tumors of the respiratory system, tumors of the digestive tract, tumors of the urinary system, tumors of male organs, tumors of female organs, skin cancer, endothelial cell tumors, brain tumors, nervous system tumors, and endocrine organ tumors.

In some embodiments, the respiratory tumors include, but are not limited to, lung and nasopharyngeal and laryngeal cancers.

In some embodiments, the gastrointestinal tumors include, but are not limited to, esophageal cancer, gastric cancer, colorectal cancer, liver cancer, pancreatic cancer, and bile duct cancer.

In some embodiments, the urinary system tumors include, but are not limited to, kidney cancer, renal pelvis and ureteral cancer, bladder cancer, and urethral cancer.

In some embodiments, the male organ tumors include, but are not limited to, penile cancer, prostate cancer, or testicular cancer.

In some embodiments, the female organ tumors include, but are not limited to, breast cancer, vulvar cancer, vaginal cancer, cervical cancer, endometrial cancer, or ovarian cancer.

In some embodiments, the nervous system tumors include, but are not limited to, astrocytoma, oligodendroglioma, ependymoma, medulloblastoma, and meningioma.

In some embodiments, the brain tumors include, but are not limited to, glioma, neurocytoma, germ-like stromal tumor, mesenchymal tumor, epithelial tumor, teratoma, and pineal tumor.

In some embodiments, skin cancers include, but are not limited to, cutaneous melanoma or non-melanoma skin cancer.

In some embodiments, the tumors are a solid tumors, including but not limited to, bladder cancer, brain cancer, breast cancer, cervical cancer, thoracic tumors, endometrial cancer, esophageal squamous cell carcinoma, gastric cancer, head tumor, pancreatic cancer, bile duct cancer, colorectal cancer, eye cancer, head and neck squamous cell carcinoma, urothelial cancer, kidney cancer, liver cancer, lymph node cancer, lung cancer, oral cancer, neck cancer, ovarian cancer, prostate cancer, testicular cancer, laryngeal cancer and uterine cancer, melanoma, salivary gland cancer, fibrosarcoma, soft tissue sarcoma and osteosarcoma. In some embodiments, the above cancers are relapsed or refractory.

In some embodiments, the breast cancer comprises: ductal carcinoma, lobular carcinoma, medullary carcinoma, glial carcinoma, tubular carcinoma, inflammatory breast cancer, triple negative breast cancer (TNBC).

In some embodiments, the ovarian cancer comprises: epithelial ovarian tumors, such as adenocarcinomas in the ovary and adenocarcinomas that migrate from the ovary into the abdominal cavity.

In some embodiments, the leukemia comprises: acute myeloid leukemia (AML), acute lymphocytic leukemia, chronic lymphocytic leukemia, chronic myeloid leukemia, hairy cell leukemia, myelodysplasia, myelodysplastic disorder, NK cell leukemia (eg, blastic plasmacytoid dendritic cell tumor), acute myeloid leukemia (AML), chronic myeloid leukemia (CML), mastocytosis, chronic lymphocytic leukemia (CLL), multiple myeloma (MM) and myelodysplastic syndrome (MDS).

In some embodiments, the pancreatic cancer is acinar cell adenocarcinoma of the pancreas, ductal cell adenocarcinoma of the pancreas, or stage IV pancreatic cancer.

In some embodiments, the lung cancer includes non-small cell lung cancer (NSCLC) and small cell lung cancer (SCLC); in some embodiments, non-small cell lung cancer (NSCLC) includes but is not limited to: squamous cell carcinoma, adenocarcinoma, large cell carcinoma; in some embodiments, the lung cancer is recurrent; in some embodiments, the lung cancer is recurrent squamous cell lung cancer or (advanced) stage IV squamous cell lung cancer.

In some embodiments, the prostate cancer is metastatic castration-resistant prostate cancer (mCRPC).

## Description of drawings

Figure 1: Schematic diagram of the structure of Nectin-4 protein.

Figure 2: Schematic diagram of the structure of Nectin-4-ADC (SWY2001-Ab1-LND 1002);
Wherein LND1002 represents the part of the linker (Linker) + drug molecule (Drug), and the circle represents the linker in the L&D is linked to the amide bond of the antibody. The Nectin-4 ADC shown in this figure is a site-specific antibody-drug conjugate, and each molecule is composed of 1 anti-Nectin-4 monoclonal antibody coupled with 1 molecule of MMAE derivative at amino acid Q295 of each heavy chain (Kabat numbering) via the linker ($NH_2$-$PEG_3$-Val-Cit). The linkage between the antibody and the linker is a stable amide bond (iso-peptide bond), and the average ratio of the drug molecule to the antibody (DAR) is 2.0.

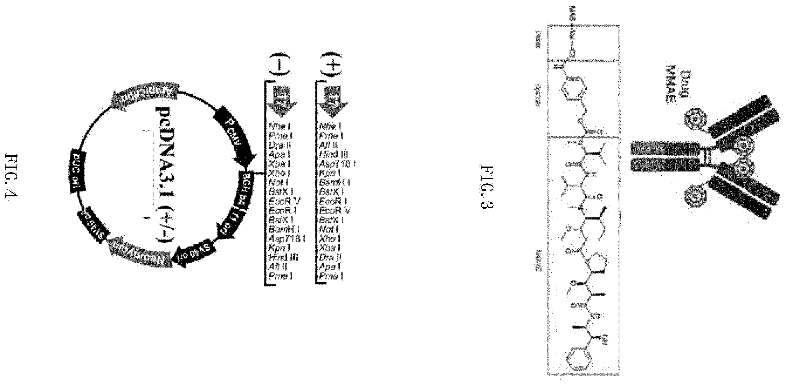

Figure 3: Schematic diagram of the structure of Nectin-4-ADC (SWY2001-Ab1-VC MMAE).

Figure 4: pcDNA3.1 map.

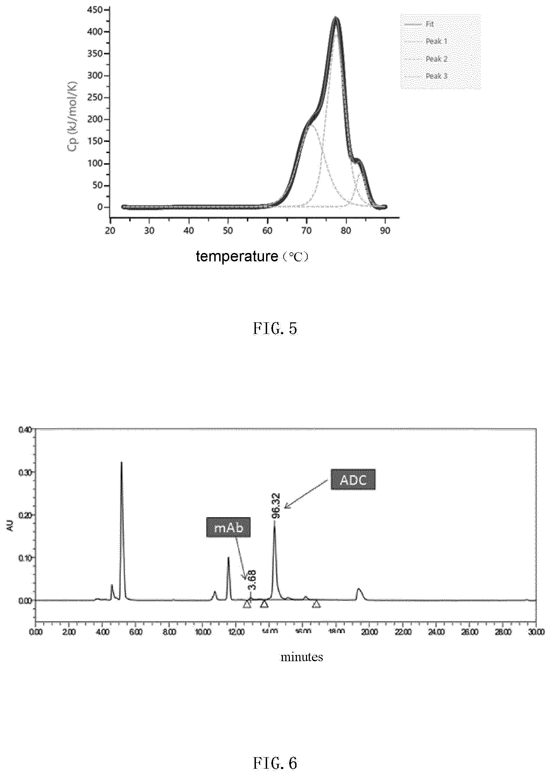

Figure 5: DSC map of humanized antibody SWY2001-Ab1.

Figure 6: Nectin-4 ADC (SWY2001-Ab1-LND1002) modification rate identification map.

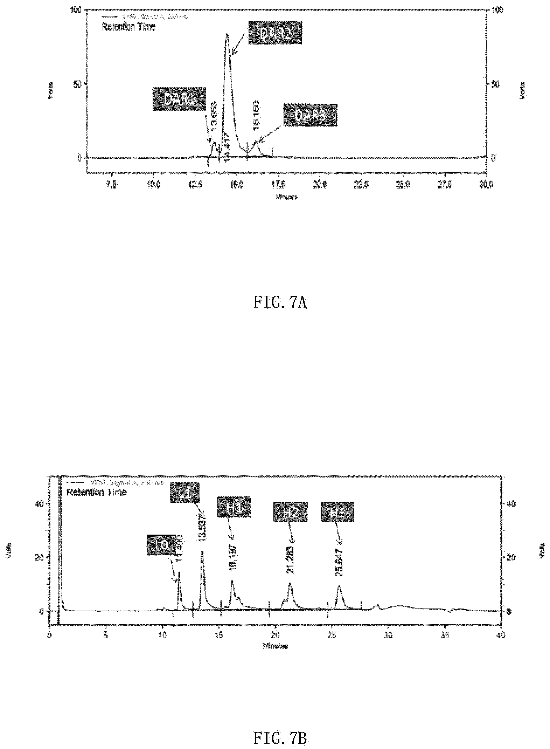

Figure 7 A: SWY2001-Ab1-LND1002 DAR value identification map.

Figure 7 B: SWY2001-Ab1-VC-MMAE DAR value identification map.

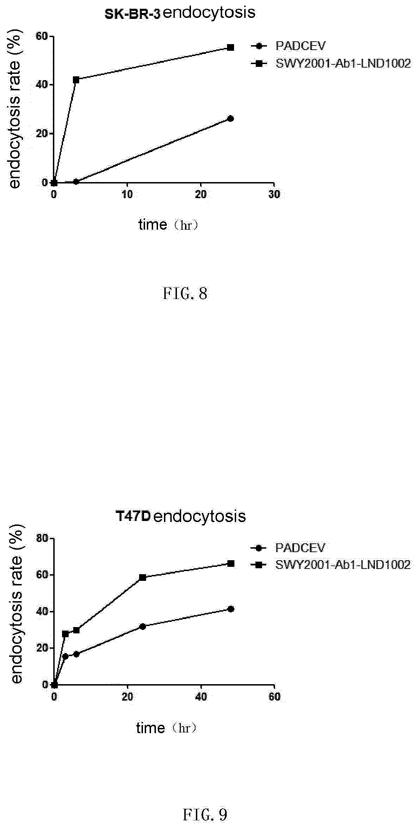

Figure 8: Experiment of endocytosis effect of SWY2001-Ab1-LND1002 in SK-BR-3 cell.

Figure 9: Experiment of endocytosis effect of SWY2001-Ab 1-LND 1002 in T47D cells.

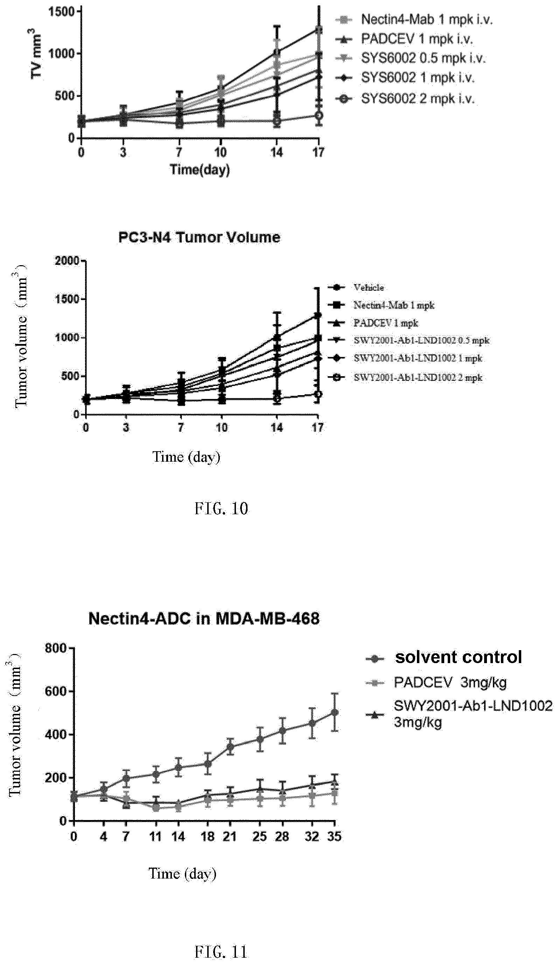

Figure 10: In vivo inhibitory effect of SWY2001-Ab1-LND1002 on mouse PC3-Nectin-4 stable-transformed tumor.

Note: mpk = mg/kg

Figure 11: In vivo inhibition experiment of SWY2001-Ab1-LND1002 on mouse MDA-MB-468 tumor.

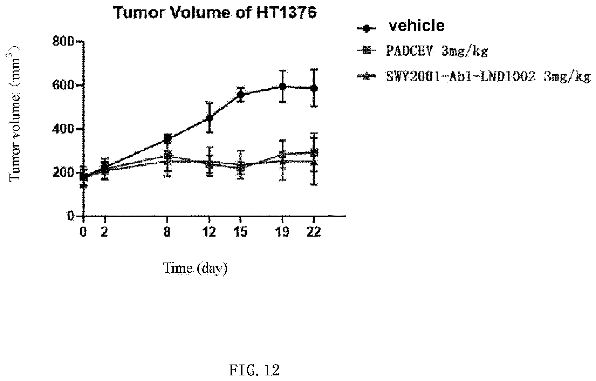

Figure 12: In vivo inhibition experiment of SWY2001-Ab 1-LND 1002 on mouse HT-1376 tumor.

## Detailed Description

Definitions:

Unless otherwise defined, all technical and scientific terms used herein have the same meaning as understood by one of ordinary skill in the art. For definitions and terms in the art, professionals can refer to Current Protocols in Molecular Biology (Ausubel). Abbreviations for amino acid residues are the standard 3- letter and/or 1 - letter codes used in the art to refer to one of the 20 commonly used L- amino acids.

Despite the numerical ranges and approximations of the parameters set forth in the broad scope of the invention, the numerical values set forth in the specific Examples are reported as precisely as possible. Any numerical values, however, inherently contain certain errors necessarily resulting from the standard deviation found in their respective measurements. Additionally, all ranges disclosed herein are to be understood to encompass any and all subranges subsumed therein. For example, a recited range of " 1 to 10" should be considered to include any and all subranges between a minimum value of 1 and a maximum value of 10, inclusive; that is, all subranges beginning with a minimum value of 1 or greater, such as 1 to 6.1, and subranges terminated with a maximum value of 10 or less, such as 5.5 to 10. Additionally, any reference referred to as "incorporated herein" should be understood to be incorporated in its entirety.

The terms "pharmaceutical composition ", "combined drug" and "drug combination" used herein are used interchangeably and refer to at least one drug and optionally a pharmaceutically acceptable carrier or combination of excipients. In certain embodiments, the pharmaceutical composition includes temporally and/or spatially separated combinations as long as they act together to achieve the objective of the present invention. For example, the components contained in the pharmaceutical composition (e.g., the antibody, nucleic acid molecule, combination of nucleic acid molecules and/or conjugates according to the present invention) may be administered to a subject as a whole, or separately. When the components contained in the pharmaceutical composition are administered to a subject separately, the components may be administered to the subject simultaneously or sequentially. Preferably, the pharmaceutically acceptable carrier is water, buffer aqueous solution, isotonic saline solution such as PBS (phosphate buffered saline), glucose, mannitol, dextrose, lactose, starch, magnesium stearate, cellulose, magnesium carbonate, 0.3% glycerin, hyaluronic acid, ethanol or polyalkylene glycols such as polypropylene glycol, triglycerides, etc. The type of pharmaceutically acceptable carrier used depends inter alia on whether the composition according to the invention is formulated for oral, nasal, intradermal, subcutaneous, intramuscular or intravenous administration. The composition according to the invention may comprise wetting agents, emulsifiers or buffer substances as additives.

The pharmaceutical composition, vaccine or pharmaceutical preparation according to the present invention may be administered by any suitable route, e.g. administered orally, nasally, intradermally, subcutaneously, intramuscularly or intravenously.

"Therapeutically effective amount" or "effective amount" used herein refers to a dose sufficient to show benefit to the subject to which it is administered. The actual amount administered, as well as the rate and time course of administration, will depend on the individual condition and severity of the subject being treated. The treatment prescription (e.g. decision on dosage, etc.) is ultimately the responsibility of general practitioners and other physicians and rely on their decisions, usually taking into account the disease being treated, the individual patient's condition, the site of delivery, the method of administration, and other known factors.

"Subject" as used herein refers to mammals, such as human, but can also be other animals, such as wild animals (e.g. herons, stork, crane, etc.), domestic animals (e.g. duck, geese, etc. ) or experimental animals (e.g. gorilla, monkey, rat, mouse, rabbit, guinea pig, marmot, ground squirrel, etc. ).

The term "antibody" refers to a whole antibody and a functional fragment thereof. "Full-length antibody" refers to a protein comprising at least two heavy (H) chains and two light (L) chains linked by a disulfide bond. Each heavy chain contains a heavy chain variable region (abbreviated as VH) and a heavy chain constant region. The heavy chain constant region contains three domains, CH1, CH2 and CH3. Each light chain contains a light chain variable region (abbreviated as VL) and a light chain constant region. The light chain constant region contains one domain, CL. The VH and VL regions can also be subdivided into regions of high variability, called complementarity determining regions (CDRs), interspersed with more conserved regions called framework regions (FRs). VH and VL each consists of three CDRs and four FRs, arranged from amino terminus to carboxy terminus in the following order: FR1, CDR1, FR2, CDR2, FR3, CDR3, FR4. The variable regions of heavy and light chains contain binding domains that interact with antigens. The constant region of an antibody mediate the binding of an immunoglobulin to tissues or factors of a host, including various cells of the immune system (e.g., effector cells) and the first component (Clq) of the classical complement system. Chimeric or humanized antibody is also encompassed in the antibody according to the present invention, the CDRs of which are encoded in the way of IMGT.

For the synthesis of antibody light and heavy chain genes, conventional genetic engineering techniques can be used. For example, the method disclosed by Chen Jianjun et al. (Chen Jianjun et al, Journal of Cellular and Molecular Immunology, 1997, Issue 3) can be referred to.

The term "humanized antibody" refers to an antibody that may comprise CDR regions derived from a human antibody and the other portions of the antibody molecule derived from one (or several) human antibodies. Furthermore, some residues of the backbone (referred to as FR) segments may be modified in order to retain binding affinity. Humanized antibodies or fragments thereof according to the present invention may be prepared by techniques known to those skilled in the art.

The term "chimeric antibody" refers to an antibody in which the variable region sequences are from one species and the constant region sequences are from another species, e.g., an antibody in which the variable region sequences are from a mouse antibody and the constant region sequences are from a human antibody. The chimeric antibody or a fragment thereof according to the present invention can be prepared by using genetic recombination techniques. For example, the chimeric antibody can be produced by cloning a recombinant DNA comprising a promoter and sequences encoding the variable regions of a non-human, especially murine monoclonal antibody according to the invention, and sequences encoding the constant regions of a human antibody. A chimeric antibody of the invention encoded by such a recombinant gene will be, for example, a murine-human chimera, the specificity of which is determined by variable regions derived from murine DNA and the isotype thereof is determined by constant regions derived from human DNA.

The term "monoclonal antibody" refers to a preparation of antibody molecules having a single molecular composition. Monoclonal antibody composition exhibits a single binding specificity and affinity for a particular epitope.

The term "bispecific antibody" is capable of binding two antigens or epitopes, respectively, and includes the light and heavy chains of an antibody that specifically binds a first antigen, and the light and heavy chains of an antibody that specifically binds a second antigen.

"Functional fragment" as used herein especially refers to an antibody fragment, such as Fv, scFv (sc refers to single chain), Fab, F(ab')$_2$, Fab', scFv-Fc fragment or diabody, or any fragment that should have increased half-life by chemical modifications, such as the addition of poly (alkylene) glycols such as polyethylene glycol ("pegylation, PEGylation" )(referred to as PEGylated fragment of Fv-PEG, scFv-PEG, Fab-PEG, F(ab')2-PEG or Fab'- PEG ) ("PEG" is polyethylene glycol), or by incorporation into a liposome. The fragment has Nectin-4 binding activity. Preferably, the functional fragment will consist of or comprise a partial sequence of the heavy or light variable chain of the antibody from which it is derived, the partial sequence being sufficient to retain the same binding specificity as the antibody from which it is derived and sufficient affinity. Such functional fragment will contain a minimum of 5 amino acids, preferably 10, 15, 25, 50 and 100 contiguous amino acids of the antibody sequence from which it is derived.

In general, for the preparation of monoclonal antibodies or functional fragments thereof, especially of murine origin, reference may be made to the technology described inter alia in the handbook "Antibodies" (Harlow and Lane, Antibodies: A Laboratory Manual, Cold Spring Harbor Laboratory, Cold Spring Harbor NY, pp.726, 1988) or to the technology described by Kohler and Milstein for preparation from hybridoma cells (Nature, 256 : 495-497, 1975).

The term "antibody drug conjugate (ADC)" or "conjugate" as used herein generally refers to an antibody or an antigen-binding fragment thereof linked with another agent such as a chemotherapeutic agent, toxin, immunotherapeutic agent, imaging probe etc. The linkage may be a covalent bond or a non-covalent interaction, e.g., by electrostatic force. To form an antibody drug conjugate, various linkers known in the art and described herein can be employed. Additionally, the antibody drug conjugate can be provided as a fusion protein that can be expressed from a polynucleotide encoding an immunoconjugate. As used herein, a "fusion protein" refers to a protein produced by linking two or more genes or gene fragments that originally encode separate proteins, including peptides and polypeptides. Translation of the fusion gene produces a single protein with functional properties from each original protein.

In a "conjugate", two or more compounds are linked together. In certain embodiments, at least some properties from each compound are retained in the conjugate. Linking can be achieved through covalent or non-covalent bonds. Preferably, the compounds of the conjugate are linked by covalent bonds. The different compounds of the conjugate can be directly bound to each other through one or more covalent bonds between the atoms of the compounds. Alternatively, the compounds can be bound to each other by chemical moieties such as linker molecules, wherein the linkers are covalently attached to atoms of the compounds. If the conjugate consists of more than two compounds, the compounds may be linked, for example, in a chain conformation, with one compound linked to the next, or several compounds each linked to a central compound.

The cytotoxic drug described herein specifically refers to a substance that inhibits or prevents cell expression activity, cell function, and/or cause cell destruction. Examples include, but are not limited to: auristatin derivatives (e.g. MMAE, MMAF ), chlortetracycline, maytansinoid and derivatives thereof (DM0, DM1, DM2, DM3, DM4 etc.), Ricin, combrestatin, Ansamitocin, calicheamicin, Duocarmycin, dolastatin and derivatives thereof, DNA topoisomerase inhibitors and their derivatives (for example: etoposide, teniposide, Dxd, SN38), Amanitin, cc1065 and its analogs, Mitomycin C, Camptothecin(CPT) and its analogs, Vincristine, Vinblastine, Colchicine, mitoxantrone, Actinomycins, Diphtheria toxin, Pseudomonas exotoxin, Abrin, Gelonin, Micronomicin, etc.

The term "immune enhancer" refers to a substance that can activate non-specific immunity and enhance the immune response of the body, such as: TLR agonist, STING agonist.

Radioisotopes include, but are not limited to: $At^{211}$, $I^{131}$, $I^{125}$, $Y^{90}$, $Re^{186}$, $Re^{188}$, $Sm^{153}$, $Bi^{212}$ or $Bi^{213}$, $P^{32}$, $Pb^{212}$, Lu.

The term "linker" refers to a structural element of a compound that enables the linkage of two compounds through one structural element of the compound and one or more other structural elements of the same compound. The linker may be a non-cleavable linker. Suitable non-cleavable linkers include, but are not limited to: $NH_2$-R-X, $NH_2NH$-R-X, and $NH_2$-O-R-X, wherein R is an alkyl or polyethylene glycol group (also known as PEG), wherein X is the active moiety. A polyethylene glycol group or PEG group may have the general formula $-(CH_2CH_2O)_n$, wherein n is an integer of at least 1. In some embodiments, n is any one of 2, 4, 6, 8, 10, 12, 16, 20, or 24.

Cleavable linkers include, but are not limited to, Lys-Phe-X, Lys-Val-Cit-PABC-X, $NH2$-$(CH_2CH_2O)_n$-Val-Cit-pABC-X and $NH2$-$(CH_2CH_2O)n$-(Val-Cit-PABC-X)$_2$, wherein X is the active moiety and n is an integer of at least 1 (such as any of 2, 4, 6, 8, 10, 12, 16, 20 or 24). PABC refers to p-aminobenzyloxycarbonyl.

By way of example only, the linker may be selected from: $NH_2$-(PEG)m-Val-Cit, $NH_2$-(PEG)m-Val-Cit-pABC, mc-Val-Cit-pABC, Val-Cit-pABC or Val-Cit, wherein m represents the number of PEG, which is an integer from 1 to 8, namely 1, 2, 3, 4, 5, 6, 7, 8.

The term "endogenous glutamine" refers to the conserved glutamine residue (Q295) at position 295 of the heavy chain of a full-length IgG antibody of human isotype, which is in close proximity to the N-Glycosylation site (N297).

Equipment and experimental materials:

Table 1: Equipment and experimental materials

| Equipment | Manufacturer | Article number |
|---|---|---|
| Human Nectin- 4 Protein, His Tag | ACRO | NE4-H52H3 |
| Cynomolgus Nectin-4 Protein, His Tag | ACRO | NE4-C52H4 |
| Mouse Nectin-4 Protein, His Tag | ACRO | NE4-M52H3 |
| Recombinant Rat Nectin-4 His-tag Protein, CF | R&D systems | 9997-N4-050 |
| SK-BR-3 | ATCC | HTB-30™ |
| 293T-Nectin - 4 | Kyinno Bio | custom made |
| PC3-Nectin - 4 | Kyinno Bio | custom made |
| HT1376 | iCell | iCell-h077 |

Example 1: Antibody acquisition and humanization

The Nectin-4 murine antibodies were prepared according to WO2022051591A2. The brief steps were as follows: Animals (BALB/c mice) were immunized with human Nectin-4-His fusion protein in adjuvant (Freund's adjuvant) by intraperitoneal injection. Animals were boosted every 2 weeks to be continuously induced until appropriate titers were developed. Blood was collected from the animals after each booster immunization, and the titer was detected by ELISA and FACS. 4 days after the last immunization, the spleens of the animals with appropriate titer were obtained, and single-cell suspension was prepared. Cells were fused with SP2/0 mouse myeloma cells using electrofusion. The fused cells were re-suspended in medium containing hybridoma cell selection agents thymidine, hypoxanthine and aminopterin (HAT), and then seeded into 96 -well plates for culture.

After 7-10 days of incubation, the culture supernatant was collected, and the clones that could bind to the human Nectin-4 protein were detected and screened by ELISA or FACS, and whether it was bound to the mouse Nectin-4 protein was verified. Hybridoma cells continue to be cultured after the addition of fresh HAT-containing medium. Two days later, the culture supernatant of the positive clones screened in the first round was collected and then tested for antibody functional activity. Afterwards, the selected positive clones were further subcloned. After successful subcloned, these antibodies were purified by conventional antibody purification methods, and the antibody variable regions were sequenced for some of the clones that met the requirements.

Nectin-4 murine antibodies obtained after animal immunization and screening were humanized. The humanization work was completed by GenScript, and three humanized antibodies SWY2001-Ab1, SWY2001-Ab2 and SWY2001-Ab3 were obtained. The sequences thereof are shown in table 2. The humanization steps were as follows:

1.1 Expression and purification of the chimeric antibodies

The variable regions of the chimeric antibodies (the chimeric antibodies were modified on the basis of the murine antibodies, and the constant regions were selected from human IgG1) were inserted into the vector pcDNA3.4 (IgG1, kappa) to construct complete heavy chain and light chain plasmids. The plasmids were then transfected into HEK293 cells, and the supernatant was collected and purified with protein A magnetic beads to obtain full-length antibodies. The solution was replaced with PBS by dialysis and desalting. The concentration and purity of the antibodies were detected by OD280 and SDS-PAGE gel electrophoresis, respectively. Then the affinities thereof were detected by surface plasmon resonance (SPR) technology.

1.2 Binding activity and affinity detection of the chimeric antibodies

The affinity detection of the chimeric antibodies was mainly determined by Biacore instrument using Surface Plasmon Resonance (SPR) technology. Detection method: The protein A chip was used to couple the binding antibodies by capturing the Fc fragments of the antibodies, and the antigen was used as the mobile phase for detection. The obtained detection data were fitted by the software provided with the instrument to determine the corresponding association constant (ka) and dissociation constant (pk) and calculate the corresponding equilibrium constant (KD).

1.3 Design of Back Mutation of Humanized Antibodies

Back mutations and combinations thereof were performed on multiple amino acid sites in the inner core regions of the light and heavy chain structures of the humanized antibodies. The corresponding light and heavy chain genes were synthesized by GenScript.

1.4 Production and affinity ranking of the back mutated humanized antibodies

After obtaining the heavy chain and light chain plasmids, the corresponding light and heavy chains were combined and paired and transfected and expressed in a 4 mL system. After the expression supernatant was obtained, SPR technology was used to detect and rank the supernatant for affinity: the protein A chip was used to capture the antibodies in the supernatant by capturing the Fc fragments of the antibodies, and the antibodies were immobilized on the sensor chip. Analyte antigen was used as the mobile phase. Surface regeneration was performed before injection of another antibody supernatant, and the process was repeated until all antibodies were analyzed. The experimental data were fitted with a 1: 1 interaction model by using Biacore analysis software. The binding-dissociation rates of the antibodies were obtained and the affinities of the antibodies were ranked by the dissociation rate constant (pk). According to the ranking results, the top 3 clones with the highest affinity were selected as candidate antibodies, and three humanized antibodies were obtained through the above steps, which were named: SWY2001-Abl, SWY2001-Ab2 and SWY2001-Ab3.

1.5 Construction, expression and affinity determination of candidate antibodies

The three candidate antibody genes were inserted into the vector pcDNA3.4 (IgG1, kappa) respectively to construct complete heavy chain and light chain antibody plasmids. The light and heavy chain plasmids were then co-transfected into Expi293F cells, and the supernatant was collected and purified with protein A magnetic beads to obtain full-length antibodies. The solution was replaced with PBS by dialysis and desalting, and the concentrations and purities of the antibodies were detected by OD280 and SDS-PAGE gel electrophoresis, respectively. Then the affinities thereof were detected by Surface Plasmon Resonance (SPR) technology.

Table 2: Relevant sequences involved in this application

| Antibody | sequence information | |
|---|---|---|
| **Antibody CDRs** | heavy chain | |
| | CDR1: GYTFTSYY | SEQ ID NO.1 |
| | CDR2: IYPGNVNT | SEQ ID NO.2 |
| | CDR3: ARGIYYFDY | SEQ ID NO.3 |
| | light chain | |
| | CDR1: QSVNND | SEQ ID NO.4 |
| | CDR2: YAS | SEQ ID NO.5 |
| | CDR3: HQDYSSPFT | SEQ ID NO.6 |
| murine antibody | VH Heavy Chain<br><br>QVQLQQSGPVLVKPGASVRISCKAS<u>GYTFTSYY</u>IHWVKQRPGQGLEWIGW<u>IYPGN</u><br><br><u>VNT</u>KYNENFRDKATLTADKSSSTSYMQLSSLTSEDSAVYFC<u>ARGIYYFDY</u>WGQGTT<br><br>LTVSS<br>SEQ ID NO.7<br>VL Light Chain<br>IIVMTQTPKFLLVSAGDRVTITCKAS<u>QSVNND</u>VAWYQEKPGQSPKLLIY<u>YAS</u>NRDTG<br><br>VPDRFTGSGYGTDFTFTISTVQAEDLAVYFC<u>HQDYSSPFT</u>FGSGTKLEIK    SEQ ID NO.8 | |
| humanized antibody SWY2001-Ab1 | SWY2001-Ab1-VH<br><br>QVQLVQSGAEVKKPGASVKVSCKAS<u>GYTFTSYY</u>IHWVRQAPGQRLEWMGW<u>IYPG</u><br><br><u>NVNT</u>KYNENFRDRVTITRDTSASTAYMELSSLRSEDTAVYYC<u>ARGIYYFDY</u>WGQGT<br><br>LVTVSS<br>SEQ ID NO.9<br>SWY2001-Ab1-VC<br>IIQMTQSPKFLSASVGDRVTITCKAS<u>QSVNND</u>VAWYQQKPGQSPKLLIY<u>YAS</u>NRDT<br><br>GVPDRFSGSGSGTDFTLTISSLQPEDFATYFC<u>HQDYSSPFT</u>FGGGTKVEI**K**<br>SEQ ID NO.10 | |

| Antibody | sequence information |
|---|---|
| humanized antibody SWY2001-Ab2 | SWY2001-Ab2-VH<br><br>QVQLVQSGAEVKKPGASVKVSCKAS<u>GYTFTSYY</u>IHWVRQAPGQRLEWMGW<u>IYPG</u><br><br><u>NVNT</u>KYNENFRDRVTITRDTSASTAYMELSSLRSEDTAVYYC<u>ARGIYYFDY</u>WGQGT<br><br>LVTVSS<br>SEQ ID NO.9 |
|  | SWY2001-Ab2-VC<br><br>AIQMTQSPSSLSASVGDRVTITCKAS<u>QSVNND</u>VAWYQQKPGKAPKLLIY<u>YAS</u>NRDT<br><br>GVPSRFSGSGSGTDFTLTISSLQAEDLAVYFC<u>HQDYSSPFT</u>FGGGTKVEIK<br>SEQ ID NO.11 |
| humanized antibody SWY2001-Ab3 | SWY2001-Ab3-VH<br><br>QVQLVQSGAEVKKPGASVKVSCKAS<u>GYTFTSYY</u>IHWVRQAPGQRLEWMGW<u>IYPG</u><br><br><u>NVNT</u>KYNENFRDRVTITRDTSASTAYMELSSLRSEDTAVYYC<u>ARGIYYFDY</u>WGQGT<br><br>LVTVSS<br>SEQ ID NO.9<br>SWY2001-Ab3-VC<br><br>AIQMTQSPSSLSASVGDRVTITCKAS<u>QSVNND</u>VAWYQQKPGKAPKLLIY<u>YAS</u>NRDT<br><br>GVPSRFSGSGSGTDFTLTISSLQPEDFATYFC<u>HQDYSSPFT</u>FGGGTKVEIK<br>SEQ ID NO.12 |

EP 4 393 951 A1

| Antibody | sequence information |
|---|---|
| Constant regions | HC<br><br>ASTKGPSVFPLAPSSKSTSGGTAALGCLVKDYFPEPVTVSWNSGALTSGVHTFPAVL<br><br>QSSGLYSLSSVVTVPSSSLGTQTYICNVNHKPSNTKVDKKVEPKSCDKTHTCPPCPA<br><br>PELLGGPSVFLFPPKPKDTLMISRTPEVTCVVVDVSHEDPEVKFNWYVDGVEVHN<br><br>AKTKPREEQYNSTYRVVSVLTVLHQDWLNGKEYKCKVSNKALPAPIEKTISKAKG<br><br>QPREPQVYTLPPSREEMTKNQVSLTCLVKGFYPSDIAVEWESNGQPENNYKTTPPV<br><br>LDSDGSFFLYSKLTVDKSRWQQGNVFSCSVMHEALHNHYTQKSLSLSPGK (SEQ ID<br><br>NO.16)<br>LC<br><br>RTVAAPSVFIFPPSDEQLKSGTASVVCLLNNFYPREAKVQWKVDNALQSGNSQESV<br><br>TEQDSKDSTYSLSSTLTLSKADYEKHKVYACEVTHQGLSSPVTKSFNRGEC (SEQ<br><br>ID NO.17) |

Example 2: Construction of Expression Vector and Expression of Humanized Antibody

The heavy and light chain DNA sequences of the humanized Nectin-4 antibodies were synthesized by Biointron, Taizhou, and the expression vector pcDNA3.1 was provided by Biointron, Taizhou (see Figure 4). The brief steps are as follows:

Design and synthesis of the heavy chain:
The synthetic heavy chain was named Nectin-4-HC. A HindIII endonuclease site was introduced at the 5' end, an EcoRI endonuclease site was introduced at the 3' end, and a Kozak sequence and a signal peptide sequence (19 amino acids) MELGLCWVFLVAILEGVQC (SEQ ID NO:14) were introduced after HindIII endonuclease site at the 5' end. The expression cassette for the heavy chain was designed as:

HindIII-Kozak sequence-signal peptide-Nectin-4-HC-stop codon-EcoRI

Design and synthesis of the light chain:
The synthetic light chain was named Nectin-4-LC. During the synthesis, a HindIII endonuclease site was introduced at the 5' end of the light chain, an EcoRI endonuclease site was introduced at the 3' end, and a Kozak sequence and a signal peptide sequence (22 amino acids): MDMRVPAQLLGLLLLWFPGSRC (SEQ ID NO: 15) were introduced after HindIII endonuclease site at the 5' end. The expression cassette for the light chain was designed as:
HindIII-Kozak sequence-signal peptide-Nectin-4-LC-stop codon-EcoRI

2) Construction of recombinant plasmids

The PCR amplification product Nectin-4-HC and the plasmid vector pcDNA3.1 were subjected to HindIII/EcoRI double digestion, ligation and transformation, and positive clones were screened by Amp+ resistance marker to confirm that the correct recombinant heavy chain expression vector was obtained. The PCR amplification product Nectin-4-LC and plasmid vector pcDNA3.1 were subjected to HindIII/EcoRI double digestion, ligation and transformation, and positive clones were screened by Amp+ resistance marker to obtain the correct recombinant light chain expression vector.

In this experiment, the expression of humanized antibodies was carried out by transiently transfecting HEK293 cells. HEK293 cells were placed in a 5% $CO_2$ incubator shaker, and incubated at 37 °C and 120 rpm with constant temperature and shaking. The cells were cultured to a density of $2.0 \times 10^6$ cells/mL, and the antibody heavy chain and light chain plasmids were added at a ratio of 0.5 mg HC and 0.5 mg LC per liter of cells. Firstly, KPM (transfection buffer) and sterile plasmids were mixed. Then, another centrifuge tube was taken, in which KPM and TA-293 transfection reagent were mixed. The transfection reagent was slowly added to the KPM mixture comprising the plasmids. The prepared plasmid-vector complex was gently mixed well, and the plasmid-vector complex was added to the cells after standing for 10 min. The cellular protein expression enhancer and transient transfection nutrient additives were added after 24 h, and the cells were harvested on the 6th day after transfection, and were purified.

Capillary isoelectric focusing (cIEF) method: 4.3 M urea, 3M urea-cIEF glue solution, cIEF MIX solution were prepared. The test product was diluted to 5 mg/mL. 234 μL of cIEF MIX solution was taken and mixed with 10 μL of sample and fully vortexed. 200 μL of the mixture was taken and transferred to the inner cannula for detection.

Monoclonal antibody size variation determination (CE-SDS) method: non-reducing sample preparation: 100 μg of the test solution was taken and SDS sample buffer was added to 95 μL. Then 5 μL of 250 mM iodoacetamide solution was added and mixed well. The reference solution was prepared in the same way. Reduction sample preparation: 100 μg of the test solution was taken and SDS sample buffer was added to 95 μL. Then 5 μL of 2-mercaptoethanol was added and mixed well. The mixed sample was incubated at 70 + 2°C for 10 min, cooled to room temperature, and centrifuged at 6000 rpm for 1 min. 80 μL of the supernatant was pipetted into the sample tube for immediate analysis. The physical and chemical properties test data are as follows:

Table 3 : Expression and physicochemical properties of Nectin-4 humanized antibody

| clone | Expression quantity (mg/L) | SEC | | | cIEF | | | CE (NR) | R CE-SDS |
|---|---|---|---|---|---|---|---|---|---|
| | | aggregation | Main peak | Fragment | Acid peak | Main peak | Basic peak | Main peak | LC+HC |
| SWY2001-Ab1 | 200.3 | ND | 100 | ND | 16.12 | 68.95 | 14.93 | 94.193 | 99.443 |
| SWY2001-Ab2 | 120.3 | 0.003 | 99.997 | ND | 9.97 | 54.94 | 35.09 | 92.769 | 98.499 |
| SWY2001-Ab3 | 185.6 | ND | 100 | ND | 31.33 | 53.89 | 14.78 | 92.651 | 98.825 |

It can be seen that the expression of the humanized antibodies obtained by the present invention were relatively high. The transient expression of the common antibody was 50-100 mg/L, and those of the three humanized antibodies of the present invention were all greater than 100 mg/L. Moreover, the expression of SWY 2001-Ab1 reached 200 mg/L. At the same time, multiple methods were used to detect the purity. The SEC-HPLC purity was more than 99%, and the reduced CD-SDS purity was more than 98%, all met the requirements of further experiments.

Example 3: DSC detection of Nectin-4 humanized antibodies

In this experiment, differential scanning calorimeter was used to detect the stability of Nectin-4 humanized antibodies. The experimental parameters were as follows. The antibody concentration was 1 mg/mL, and the loading volume was 325 μL. The experimental results are shown in Figure 5. The specific DSC parameter settings were as follows:

Table 4: DSC parameter settings

| Parameter category | parameter value |
| --- | --- |
| start temperature of the scan (°C) | 20 |
| termination temperature of the scan (°C) | 90 |
| rate of the scan (°C/h) | 60 |
| cleaning between samples | 14% Decon90, Water |
| signal feedback mode | None |
| analysis software version | 1.40 |

It can be seen from the results in Figure 5 that the antibodies obtained in this application have good stability.

Example 4: Affinity and Species Cross Detection of Nectin-4 Humanized Antibodies

In this experiment, Fortebio was used to detect the affinity of Nectin-4 humanized antibodies to human Nectin-4 protein. After the Nectin-4 protein was incubated with the anti-Nectin-4 antibody sample, the signal value was detected by Fortebio to analyze the affinity of the sample to human Nectin-4. The experimental results are shown in Table 5, showing that the affinities of the three humanized antibodies to the human Nectin-4 protein did not substantially decrease after the humanization was completed. The chimeric antibodies were engineered on the basis of mouse-derived antibodies, and the constant region is selected from human IgG1.

Table 5: Affinity of anti-Nectin-4 antibodies to human Nectin-4 protein

| Sample ID | KD (M) | kon(1/Ms) | kdis(1/s) | Full R^2 |
| --- | --- | --- | --- | --- |
| Chimeric antibody | 2.80E-09 | 6.46E+05 | 1.81E-03 | 0.9974 |
| SWY2001- Ab1 | 5.92E-09 | 7.56E+05 | 4.47E-03 | 0.9909 |
| SWY2001- Ab2 | 5.51E-09 | 7.52E+05 | 4.14E-03 | 0.9939 |
| SWY2001- Ab3 | 5.08E-09 | 8.36E+05 | 4.25E-03 | 0.9937 |

The affinities of Nectin-4 humanized antibodies to Nectin-4 proteins of different species were performed by ELISA. Nectin-4 proteins of different species (human Nectin-4, cynomolgus Nectin-4, rat Nectin-4, mouse Nectin-4) were incubated with Nectin-4 humanized antibody samples of different concentrations, and then incubated with the secondary antibody which binds IgG (Goat anti-Human IgG (H+L) Cross-Adsorbed Secondary Antibody. The signal values of different concentrations were detected by ELISA to analyze the affinities of the samples to Nectin-4 proteins of different species. The experimental results are shown in Table 6, showing that the Nectin-4 humanized antibodies have good affinity for human, rat and cynomolgus Nectin-4 proteins.

Table 6: Species Cross Reactivity of Nectin-4 Humanized Antibodies

| Sample          EC50 | Human ng/mL | Cynomolgus ng/mL | rat ng/mL | mouse ng/mL |
|---|---|---|---|---|
| PADCEV | 0.877 | 0.968 | 1.01 | 82.4 |
| SWY2001- Ab1 | 1.21 | 0.647 | 0.857 | 674000 |
| SWY2001- Ab2 | 1.13 | 0.646 | 0.726 | 1050000 |
| SWY2001- Ab3 | 1.02 | 0.832 | 0.942 | 2210000 |

Example 5 : Preparation of Nectin-4 Antibody Drug Conjugate

1. SWY2001-Ab1-LND1002 enzymatic coupling:

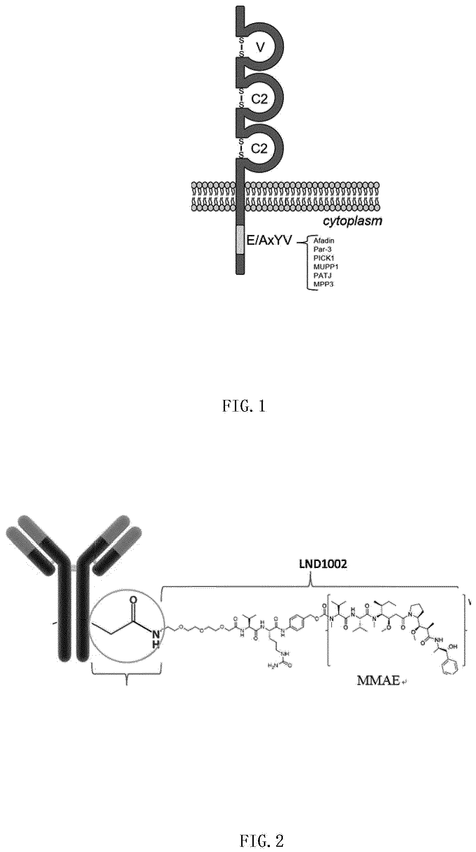

LND1002 (dissolved in DMSO at 1g: 5 mL, the structure is shown in Figure 2), 10x reaction buffer, the anti-Nectin-4 antibody, mTgase (a transglutaminase which catalyzes the transglutaminase reaction at the acceptor glutamine near the N-glycosylation site, and can specifically catalyze the transglutaminase reaction at Kabat numbering Q295) and 20% $H_2O$ were added to the EP tube in sequence, sealed and mixed, and then placed at 30°C. The reaction time did not exceed 72 h. When the coupling rate was 95%, the reaction was terminated and purified immediately. The reaction conditions were as follows:

Table 7 : SWY2001 -Ab1-LND1002 coupling reaction system

| batch | Mab-MMAE |
|---|---|
| reaction scale | 10 mg |
| Antibody Concentration | 10 mg/mL |
| Drug to Antibody molar ratio | 30:1 |
| mTgase | 0.6 mg/ mL |
| Buffer | 50 mM Tris-OAc + 2 mM EDTA + 0.02% PS20 pH 8.0 |
| Reaction conditions | 30°C 72 h |
| DAR | 2.039 |

According to the experimental results, it was confirmed that the transglutaminase catalytic site of the antibody of the present application is at the Q 295 position near the N- glycosylation site.
The transglutaminase sequence used was as follows (SEQ ID NO: 13) :

DSDERVTPPAEPLDRMPDPYRPSYGRAETIVNNYIRKWQQVYSHRDGRKQQMTEEQR
EWLSYGCVGVTWVNSGQYPTNRLAFAFFDEDKYKNELKNGRPRSGETRAEFEGRVAK
DSFDEAKGFQRARDVASVMNKALENAHDEGAYLDNLKKELANGNDALRNEDARSPF
YSALRNTPSFKDRNGGNHDPSKMKAVIYSKHFWSGQDRSGSSDKRKYGDPEAFRPDR
GTGLVDMSRDRNIPRSPTSPGESFVNFDYGWFGAQTEADADKTVWTHGNHYHAPNGS
LGAMHVYESKFRNWSDGYSDFDRGAYVVTFVPKSWNTAPDKVTQGWP

2. SWY2001-Ab1-VC-MMAE chemical coupling:

The antibody was replaced in PBS buffer containing 5 mM EDTA, pH=6.0 to make the antibody concentration of 10 mg/mL. 3 molar equivalents of TCEP reducing agent, and 1/50 volumes of 1 M dipotassium hydrogen phosphate were added according to the amount-of-substance concentration of the antibody (SWY2001-Ab1). The reaction was heated in a water bath at 37°C for 1 h, then added with 6 molar equivalents of MC-VC-pABC-MMAE, and 80 μL DMSO per 1 mL volume. The reaction was at room temperature for 30 min. 12 molar equivalents of L -cysteine was added, and the reaction was stopped after 20 min, and the final product was formed. The antibody was linked to the linker-drug through the sulfhydryl group on its inter-chain cysteine. The structure is shown in Figure 3.

Example 6: Analysis and identification of physical and chemical properties of SWY2001-Ab1-ADC

1. Identification of modification rate of enzymatically coupled SWY2001-Ab1-LND1002

Experimental steps:

1) Sample treatment: SWY2001-Ab1-LND1002 72 h modification solution was treated with 50 mM ammonium acetate, 20 mM DTT, 55 mM Tris-HCl buffer for 30 min at 30°C + 2°C, and centrifuged at 12,000 rpm for 5 min.

2) Loading: 10 μL (30 μg ) of supernatant was taken and added to a chromatographic column (waters xbridge C4, 3.5 μm, 4.6 mm*250 mm)

3) Elution: Mobile phase A solution was 0.1% TFA aqueous solution. Mobile phase B solution was 0.1% acetonitrile solution. The ratio of mobile phase A solution: B solution was adjusted at 0, 5, 8, 15, 20, 22, 25, 30 min respectively to 9:1, 7:3, 6.5:3.5, 6:4, 5.5:4.5, 5:5, 1:9, 9:1 elution. The flow rate was controlled at 0.8 mL/min, the column temperature was 60°C, and the detection wavelength was 280/254 nm.

The experimental results are shown in Fig. 6, showing that the modification rate of SWY2001-Ab1-LND1002 reaction for 72 h was 96.32%.

2. Detection of DAR value of SWY2001-Abl-ADC

Experimental steps:

Reversed-phase chromatography (RP-HPLC) was used to determine the drug-antibody coupling ratio, DAR value of the product, referring to "Chinese Pharmacopoeia" 2020 Edition, Part Four, 0512 High Performance Liquid Chromatography.

Experimental equipment: high performance liquid chromatograph Agilent 1260
Column: PLRP-S 1000A, 5um, 50*2.1mm
Mobile phase: Mobile phase A: 0.1% (v/v) TFA aqueous solution
Mobile phase B: 0.1% (v/v) TFA in acetonitrile

The detection method was as follows:

| | |
|---|---|
| Flow rate | 0.25mL/min |
| Detection wavelength | 280nm |
| Injection volume | 10μL |
| Column oven temperature | 80°C |

The gradient elution procedure was as follows:

| time (min) | Mobile phase A solution (%) | Mobile phase B solution (%) |
|---|---|---|
| 0 | 73 | 27 |
| 3 | 73 | 27 |
| 8 | 65 | 35 |
| 25 | 57 | 43 |
| 26 | 5 | 95 |

(continued)

| time (min) | Mobile phase A solution (%) | Mobile phase B solution (%) |
|---|---|---|
| 31 | 5 | 95 |
| 31.5 | 73 | 27 |
| 40 | 73 | 27 |

The experimental results were analyzed using the area normalization method.

The calculation results were as follows:

$$\text{DAR average} = \text{DAR0\%} \times 0 + \text{DAR1\%} \times 1 + \text{DAR2\%} \times 2 + \text{DAR3\%} \times 3$$

The experimental results are shown in Figure 7 (Figure 7A and Figure 7B) and Table 8.

Table 8: DAR values of SWY2001-Abl-ADC

| sample name | SWY2001- Ab1-LND1002 | SWY2001- Ab1-VC - MMAE |
|---|---|---|
| DAR | 2.039 | 4.176 |

Example 7: Endocytosis of SWY2001-Ab1-LND1002

Experimental steps:

SK-BR-3 cells and T47D cells (human breast cancer cells) were collected and re-suspended in culture medium. The re-suspended target cells were gently blew several times into single cell suspension, and cell viability and cell count were determined by trypan blue staining. The cell density was adjusted to $1 \times 10^5$ cells/mL. The cells were seeded in a confocal 96-well plate cell culture dish at 100 $\mu$L/well, and the number of cells seeded in each well was $1 \times 10^4$. ADCs labeled with Zenon™ pHrodo™ iFL were added to a 96-well plate at a final concentration of 2 $\mu$g/mL, which was then placed in a 37°C, 5% $CO_2$ incubator for continuous incubation for 24 hours. All images were observed and captured with a 20X objective of a laser confocal microscope.

The experimental results are shown in Figures 8 and 9. SWY2001-Ab1-LND1002 was endocytosed into cells and localized to lysosomes with an acidic environment, and its endocytosis rate was much higher than that of the drug PADCEV. For antibody-drug conjugates, in general, when the endocytosis rate is fast and the drug has a strong ability to enter tumor cells, it can release toxins better, and show effect faster. Therefore, the ability of the antibody-drug conjugate obtained in this application to enter tumor cells is significantly better than that of PADCEV.

Example 8: Inhibitory effect of SWY2001-Abl-LND1002 on the growth of different cells in vitro

Experimental steps:

The target cells were collected and re-suspended into single cell suspension. The cell viability and cell count were determined by trypan blue staining. The cell density was adjusted to $1 \times 10^5$ cells/mL. The cells were added to a 96-well black flat-bottom cell culture plate at 100 $\mu$L per well. The diluted test product was added at 20 $\mu$L/well to the 96-well black flat-bottom cell culture plate that has been seeded with cells, which was then placed in a cell incubator (37 °C, 5% $CO_2$) and incubated for $66\pm3$ hr. Resazurin sodium solution (0.03%) was added at 20 $\mu$L per well and reacted for 3-4 h at 37 °C. The fluorescence value was read with a microplate reader at 550 nm/610 nm. Prism or similar graphing software was used to draw a graph and fit the half inhibitory concentration (IC50) of the reference standard and the sample. The output parameter C was $IC_{50}$ in ng/mL.

The experimental results are shown in Table 9, showing that SWY2001-Ab1-LND1002 could inhibit the growth of 293T-Nectin-4, SK-BR-3 and PC3-Nectin-4 cancer cells in vitro. The killing data of 293T-Nectin-4 stable transformed strain in vitro showed that SWY2001-Ab1-LND1002 was slightly better than PADECV The killing data of SK-BR-3 in vitro showed that the inhibition of SWY2001-Ab1-LND1002 was 4 times higher than that of PADCEV The killing data of PC3-Nectin-4 in vitro showed that SWY2001-Ab1-LND1002 was not significantly different from PADCEV.

Table 9: In vitro inhibitory effects of SWY2001-Ab1-LND1002 on several cancer cells

| Cell | SWY2001- Ab1-LND1002 (IC50 ng/mL) | PADCEV (IC50 ng/mL) |
|---|---|---|
| 293T-Nectin - 4 | 2.6789 | 3.8262 |
| SK-BR-3 | 334.62 | 1487.85 |
| PC3-Nectin-4 | 40.14 | 34.15 |

Example 9: In vivo efficacy of SWY2001-Ab1-LND1002

**1. Drug efficacy experiment as to inhibition of prostate cancer in vivo**

In this experiment, age-appropriate female NUNU mice were inoculated with PC3-Nectin-4 stably transformed cells (a nude mouse model transplanted with human prostate cancer, having high expression of Nectin-4). When the tumor volume grew to about 190mm$^3$ (d19 after inoculation), 42 animals with good tumor growth were selected and equally divided into 6 groups according to tumor volume (D0), i.e., vehicle control group, Nectin4-mab 1 mg/kg group, PADCEV 1 mg/kg group, SWY2001-Ab1-LND1002 0.5, 1 and 2 mg/kg groups, 7 mice in each group. After administration, the mice were weighed, and the data were recorded. By measuring the tumor diameter at different times after administration, the growth of the tumor was dynamically observed. On day 17 (the 17th day after administration), the experiment was over. After the mice were asphyxiated with carbon dioxide, the tumors were removed and weighed.
Under the experimental conditions (see Figure 10), SWY2001-Ab1-LND1002 could inhibit tumor growth in a dose-dependent manner (P < 0.05). Under the condition of 1 mg/kg dose, the tumor inhibition ratio of SWY2001-Ab1-LND1002 was significantly better than that of PADCEV (58.3% vs 39.0%). The inhibition ratios of the tumor weight in Nectin4-mab 1 mg/kg, PADCEV 1 mg/kg, SWY2001-Ab1-LND1002 0.5, 1 and 2 mg/kg groups were 28.2%, 39.0%, 22.1%, 58.3%, and 79.4% respectively.

**2. Drug efficacy experiment as to inhibition of breast cancer in vivo**

In this experiment, nude mice were inoculated with MDA-MB-468 cells to construct a nude mouse model of human breast cancer MDA-MB-468 xenografts. When the tumor volume grew to about 100 mm$^3$ (d25 after inoculation), 16 animals with good tumor growth were selected and divided into 3 groups according to tumor volume (D0), 8 in the vehicle control group and 4 in each of the other experimental groups, which were intravenously given 0.9% sodium chloride injection (0.9% INJ NS (normal saline), vehicle control group), and drugs PADCEV and SWY2001-Ab1-LND1002 3 mg/kg (single administration) respectively. After administration, the mice were weighed, and the data were recorded. By measuring the tumor diameter at different times after administration, the growth of the tumor was dynamically observed. At the end of the test (D29), the mice were asphyxiated with carbon dioxide, and the tumors were removed and weighed.
The experimental results are shown in Figure 11. Under the experimental conditions, the inhibition ratios of the PADCEV 3 mg/kg group and the SWY2001-Ab1-LND1002 3 mg/kg group were 72.3% and 66.3%, respectively. Compared with the vehicle control group, the tumor growth was significantly inhibited (P<0.001).
**3. Drug efficacy experiment as to inhibition of bladder cancer in vivo** In this experiment, age-appropriate female NUNU mice were inoculated with HT-1376 cells, each inoculated with 5 × 10$^6$ cells. When the tumor volume grew to about 100 mm$^3$ (d21 after inoculation), 12 animals with good tumor growth were selected, and divided evenly into 3 groups according to tumor volume (D0). A: 4 mice in the vehicle control group, given 0.9% sodium chloride injection (0.9% INJ NS (physiological saline), vehicle control group); B: 4 mice in the PADCEV experimental group, PADCEV 3 mg/kg (single administration); C: 4 mice in the SWY2001-Ab1-LND1002 experimental group, given 3 mg/kg (single administration). The mice were weighed after administration, and the data were recorded. By measuring the tumor diameter at different times after administration, the growth of the tumor was dynamically observed. At the end of the test (D22), the mice were asphyxiated with carbon dioxide, and the tumors were removed and weighed.
The experimental results are shown in Figure 12. Under the experimental conditions, the tumor inhibition ratios of PADCEV 3 mg/kg and SWY2001-Ab1-LND1002 3 mg/kg were 42.1% and 49.4%, respectively. Compared with the vehicle control group, the Nectin-4-ADC 3mg/kg (single administration) group can significantly inhibit the growth of tumor. Compared with the positive control PADCEV 3mg/kg (single administration) group, the tumor inhibition ratio was significantly improved in the SWY2001-Ab1-LND1002 3mg/kg group.

Example 10 :Safety evaluation study of SWY2001-Ab1-ADC

10.1 Test comparing the effect of coupling SWY2001-Ab1-VC-MMAE with a chemical method

In this experiment, 6 male cynomolgus of appropriate age were selected, and the toxic reaction was observed by intravenous injection of the reference product and the test product SWY2001-Ab1-ADC. The dosage design of the test product SWY2001-Ab1-LND1002 (DAR2) and the reference product SWY2001-Abl-VC-MMAE (DAR4) is shown in the table below. The products were administered by intravenous injection once a week for 2 weeks. After the second dose, the subjects were observed for 7 day (general observation twice a day and careful observation once a day). Body weight changes were observed at D1 before administration, D7 and D14 after administration, and hematological and blood biochemical indexes were detected.

Table 10: Toxicity Experiment Design Protocol

| group | Dosage (mg/kg/day) | Concentration (mg/kg) | Number of animals |
|---|---|---|---|
| control group | 6 | 5 | 2 |
| Test product low-dosage group | 6 | 5 | 2 |
| Test product high-dosage group | 9 | 5 | 2 |

The experimental results are shown in the following table:

Table 11: Toxicity test results

| Observed Indexes | Reference product SWY2001-Ab1-VC-MMAE stock solution (DAR4) 6 mg/kg | Test product SWY2001-Ab1-LND1002 stock solution ( DAR2 ) | |
|---|---|---|---|
| | | 6 mg/kg | 9/12 mg/kg ( Adjusted to 12mg/kg for the third dose ) |
| Clinical Observation | 9861# : The left hind paw was ulcerated from D8 (the ulcerated area increased at D9), the left hind limb was slightly swollen from the knee joint to the ankle joint, the areas around the eyes were discolored, and the forelimbs were desquamated. D15, severe weakness of the left hind limb, ulceration in many parts of the limbs, desquamation all over the body, discoloration around the mouth, nose and face. 9862#: The left hind limb was ulcerated from D8 (the area of the ulceration increased with time), and the stools were yellow and loose. D15 The left hind limb of the animal was slightly swollen below the knee joint, the middle of the tail was ulcerated, and the animal was mildly wasting. Mild weakness of the limbs was seen about 4 hours after the administration. D16 (after the 3rd dose), death was found. A smaller thymus was observed by gross anatomical observation. | 9863# : **D11 red eye sockets;** D14 the animal had five ulcers at the ankle joint of the left hind limb. 9864# : **D11 red eye sockets.** | 9865# : **D15-D16 red** eye sockets, desquamation of both hind limbs, overall desquamation at the administration site. 9866#: **D9 redness around both** eyes. D15-D16 desquamation of both hind limbs, overall desquamation at the administration site. |
| weight gain | 9861# : D14-D10 increased by 0.72% 9862# : D14-D10 decreased by 6.6% | 9863# : D14-D10 reduced by 2.5% 9864# : D14-D10 reduced by 3.6% | 9865# : D14-D10 increased by 5% 9866# : D14-D10 reduced by 3.8% |

(continued)

| Observed Indexes | Reference product SWY2001-Ab1-VC-MMAE stock solution (DAR4) 6 mg/kg | Test product SWY2001-Ab1-LND1002 stock solution ( DAR2 ) | |
| --- | --- | --- | --- |
| | | 6 mg/kg | 9/12 mg/kg ( Adjusted to 12mg/kg for the third dose ) |
| ophthalmologic examinations | 9861#: **D19 The eyelids of both eyes were swollen and the mucous membrane of the eyelids was red.** | Animals were not given the 3rd dose, and no ophthalmologic examination was performed. | **D19 No abnormality was found in both animals.** |

The above experimental results showed that: in general, according to clinical observations, the skin toxicity and ocular toxicity of the reference product were more serious than those of the test product at the same dosage. Specifically, only mild ocular toxicity (redness around both eyes) was observed for the test product SWY2001- Ab 1-LND 1002 at high dosage (9 mg/kg) . In contrast, at the low-dosage of 6 mg/kg, the control group not only showed symptoms of ocular toxicity, but also showed left hind paw ulceration, slightly swollen from the knee joint to the ankle joint of the left hind limb, discoloration around both eyes, desquamation of both forelimbs and other serious toxic reactions, and even serious adverse reactions such as animal death after the third dose. Even if the test product was adjusted to 12 mg/kg in the third dose (much higher than the dosage of the reference product, 6mg/kg), no abnormality was found in both animals, and the safety was significantly better than that of the reference product.

10.2 Cynomolgus safety experiment comparing with PADCEV (commercially available)

The experiment used 10 cynomolgus monkeys, half male and half female, which were randomly divided into 5 groups with 2 animals in each group. The first group was given 6 mg/kg commercial control product (PADCEV®), and the second to fifth groups were given the test product SWY2001-Ab1-LND1002 by intravenous infusion. The administration amount was 10 mL/kg, and the infusion rate was 0.5 mL/kg/min. The dosage, concentration, frequency and cycle of each group of animals are shown in the following table:

Table 12: Toxicity Experiment Design Protocol

| group | Test product /control product | dosage (mg/kg) | concentration (mg/mL) | Administration frequency and cycle |
| --- | --- | --- | --- | --- |
| 1 | commercial control product | 6 | 0.6 | Dosing once a week for a total of 5 doses CD1, D8, D15, D22, D29) |
| 2 | Test product | 6 | 0.6 | |
| 3 | Test product | 12 | 1.2 | |
| 4 | Test product | 18 | 1.8 | |
| 5[a] | Test product | 15/18 | 1.5/1.8 | Dosing once every 2 weeks for a total of 3 doses (D1, D15, D29) |
| a:Starting on D15, the dosage was adjusted to 18 mg/kg | | | | |

At the same dosage (6 mg/kg), the test product and the commercial control product have basically the same toxicity signs, including skin abnormalities, white blood cells and differential counts, red blood cell-related indicators (RBC, HGB, HCT), AST, ALT, PLT and FIB abnormalities, abnormal corneal histopathology. The commercial control product exhibited earlier abnormal symptoms than the test product (D7 vs D14) and more severe skin toxicity (desquamation vs ulceration).

FDA data of the commercial control product showed that: in a 4-week long-term toxicity test of cynomolgus monkeys with repeated administrations, 1, 3 and 6 mg/kg of the commercial control product were given weekly, wherein 3 animals died in the early stage of the test (D11). Major toxicities included skin lesions, bone marrow toxicity, and mild hepatotoxicity, and all animals at 6 mg/kg were discontinued after the second dose (D8) due to severe toxicity signs.

It can be seen that the test product at the same dosage had less toxicity risk than the commercial control product PADCEV®.

Based on the above experimental results, it can be seen that the overall effect of the antibody drug conjugate obtained in this application is significantly better than that of PADCEV in endocytosis, in vitro tumor cell inhibition experiments, and in vivo tumor inhibition efficacy experiments (prostate cancer, breast cancer and bladder cancer). In particular, the preliminary test of safety evaluation shows that the antibody drug conjugate obtained in this application has a wider therapeutic window, less side effects, and no obvious adverse reactions such as skin toxicity and ocular toxicity.

Example 11 : SWY2001 -Ab1-LND1002 stability test

**Plasma Stability Experiment**

The stability of SWY2001-Abl-LND1002 and MMAE in SD rat, cynomolgus and human plasma was determined by in vitro plasma incubation at 37°C. In addition, PBST was selected as the negative control group to examine the reliability of the entire test system. Among them, the incubation sample of each incubation group was collected at 0 h, 24 h, 48 h (D2), 72 h (D3), 96 h (D4), 168 h (D7) and 336 h (D14), respectively. The concentration of MMAE in plasma of various species and PBST was determined by LC-MS/MS method. The results of the average generation percentage of MMAE after incubation of SWY2001-Ab1-LND1002 in plasma of various species and PBST are shown in Table 12.

Table 13 The average generation percentage (%) of MMAE in the plasma of various species

| Incubated substance | Species | Incubation concentration μM | The average generation percentage (%) of MMAE | | | | | | |
|---|---|---|---|---|---|---|---|---|---|
| | | | Time of incubation (h) | | | | | | |
| | | | 0 | 24 | 48 | 72 | 96 | 168 | 336 |
| ADC | human | 0.02 | 0.00 | 0.00 | 0.10 | 0.13 | 0.21 | 0.47 | 0.82 |
| | | 0.2 | 0.00 | 0.05 | 0.10 | 0.16 | 0.22 | 0.43 | 0.93 |
| | | 2 | 0.00 | 0.05 | 0.10 | 0.17 | 0.22 | 0.48 | 1.00 |
| | cynomolgus | 0.02 | 0.00 | 0.00 | 0.11 | 0.19 | 0.24 | 0.44 | 0.72 |
| | | 0.2 | 0.00 | 0.06 | 0.11 | 0.19 | 0.26 | 0.47 | 0.72 |
| | | 2 | 0.00 | 0.05 | 0.12 | 0.19 | 0.26 | 0.52 | 0.75 |
| | rat | 0.02 | 0.00 | 0.06 | 0.14 | 0.22 | 0.29 | 0.45 | 0.97 |
| | | 0.2 | 0.00 | 0.07 | 0.15 | 0.24 | 0.30 | 0.55 | 1.20 |
| | | 2 | 0.00 | 0.07 | 0.14 | 0.23 | 0.40 | 0.74 | 1.04 |
| | PBST | 0.02 | 0.00 | 0.00 | 0.00 | 0.14 | 0.10 | 0.18 | 0.20 |
| | | 0.2 | 0.00 | 0.03 | 0.05 | 0.08 | 0.10 | 0.18 | 0.24 |
| | | 2 | 0.00 | 0.02 | 0.05 | 0.08 | 0.11 | 0.18 | 0.25 |

The experimental results showed that: within the tested concentration range, after three concentrations of antibody-drug conjugates were incubated in human, monkey, and rat plasma for 336 hours, the amount of the produced small molecule MMAE accounted for about 1% of its theoretical amount, confirming that it has good stability.

The above descriptions are of preferred embodiments only, which serve as examples only and do not limit the combination of features necessary to carry out the invention. The headings provided are not intended to limit the various embodiments of the invention. Terms such as "comprising", "comprises" and "including" are not intended to be limiting. In addition, the absence of a numeral modifier includes the plural, and "or" means "and/or", unless stated otherwise. Unless otherwise defined herein, all technical and scientific terms used herein have the same meaning as commonly understood by one of ordinary skill in the art.

All publications and patents mentioned in this application are incorporated herein by reference. Various modifications and variations of the described methods and compositions of this invention will be apparent to those skilled in the art without departing from the scope and spirit of this invention. While the invention has been described in terms of specific preferred embodiments, it should be understood that the invention as claimed should not be unduly limited to these embodiments. Indeed, various modifications of the described modes for carrying out the invention that are obvious to those skilled in the relevant art are intended to be included within the scope of the appended claims.

## Claims

1. An antibody drug conjugate (ADC) capable of specifically binding to Nectin-4, wherein the antibody or a functional fragment thereof comprises a heavy chain variable region and a light chain variable region, wherein the heavy chain variable region sequence is the amino acid sequence shown in SEQ ID NO.9; and the light chain variable region sequence is selected from the amino acid sequences shown in SEQ ID NO. 10-12.

2. The antibody drug conjugate according to claim 1, wherein the heavy chain variable region sequence of the antibody or the functional fragment thereof is an amino acid sequence shown in SEQ ID NO.9 or a sequence having at least 80%, 85%, 90%, 95%, 96%, 97%, 98% or 99% sequence identity thereto, and the light chain variable region sequence is selected from amino acid sequences shown in SEQ ID NOs.10-12 or a sequence having at least 80% , 85%, 90%, 95%, 96%, 97%, 98% or 99% sequence identity thereto.

3. The antibody drug conjugate according to claim 1 or 2, wherein the antibody is a monoclonal antibody.

4. The antibody drug conjugate according to any one of claims 1-3, wherein the antibody is a humanized antibody.

5. An isolated polynucleotide encoding a light chain of an antibody or functional fragment thereof according to any one of claims 1-4 and/or a heavy chain of an antibody or functional fragment thereof according to any one of claims 1-4; or encoding an antibody or a functional fragment thereof according to any one of claims 1-4.

6. An expression vector comprising the polynucleotide according to claim 5 operably linked to a regulatory sequence which allows expression of a polypeptide encoded thereby in a host cell or in a cell-free expression system.

7. A host cell comprising the expression vector of claim 6.

8. A conjugate comprising an antibody or a functional fragment thereof conjugated to one or more drugs selected from cytotoxic drugs, immune enhancers and radioisotopes, preferably, the drug is selected from auristatin derivatives, maytansinoid derivatives, camptothecin analogs, DNA topoisomerase I inhibitors and derivatives thereof, most preferably the drug is selected from MMAE, MMAF, DM1, DM4, DXD, and SN38 and derivatives thereof, and, wherein the antibody is the antibody or functional fragment thereof according to any one of claims 1-4.

9. The conjugate according to claim 8, wherein the drug is conjugated to the antibody or functional fragment thereof through a linker, and the linker is linked to the antibody or functional fragment thereof through a thiol group or an amino group, and the linker is selected from mc-Val-Cit-pABC, mc-Val-Cit, $NH_2$-(PEG)m-Val-Cit, and $NH_2$-(PEG)m-Val-Cit-pABC, wherein m is an integer from 1 to 8.

10. The conjugate according to claim 8, wherein the conjugate has the structure Ab-(L-U)$_n$, wherein Ab represents the antibody or functional fragment thereof, L represents a linker, U represents the drug, and n is an integer or a decimal from 1 to 8.

11. The conjugate according to any one of claims 8 to 10, wherein the antibody or functional fragment thereof is a monoclonal antibody or a bispecific antibody, preferably a humanized antibody, most preferably a fully human antibody.

12. The conjugate according to any one of claims 8-11, wherein the antibody or functional fragment thereof is of the IgG isotype, preferably an IgG1 antibody.

13. A pharmaceutical composition comprising the conjugate of any one of claims 8 to 12, and a pharmaceutically acceptable carrier.

14. Use of the conjugate according to any one of claims 8 to 12 or the pharmaceutical composition according to claim 13 in the manufacture of a medicament for the treatment or prevention of a tumor.

15. The use according to claim 14, wherein the tumor is a Nectin-4 positive tumor, preferably a Nectin-4 positive solid tumor, preferably selected from prostate cancer, gastric cancer, esophageal cancer, pancreatic cancer, breast cancer, bladder cancer, lung cancer, ovarian cancer, colon cancer, liver cancer, head and neck cancer and gallbladder cancer, particularly preferably said tumor is prostate cancer, breast cancer, bladder cancer, lung cancer, ovarian cancer; most preferably prostate cancer, breast cancer, or bladder cancer.

16. A medical preparation comprising the antibody-drug conjugate according to any one of claims 8 to 14.

17. The medical preparation according to claim 16, wherein the preparation is in the form of a kit, comprising a container comprising the antibody-drug conjugate according to any one of claims 9-13.

18. Use of the conjugate according to any one of claims 8-12 and an anti-proliferative agent in the manufacture of an anti-tumor medicament.

19. A pharmaceutical composition comprising the conjugate according to any one of claims 8-12 and an anti-proliferative agent.

20. A method of treating a tumor in a subject, comprising administering to the subject an effective amount of the antibody drug conjugate according to any one of claims 8-12 or the pharmaceutical composition according to claim 13, or the medical preparation according to claim 16.

21. A method of treating a tumor in a subject, comprising administering to the subject an effective amount of the antibody drug conjugate according to any one of claims 8-12 or the pharmaceutical composition according to claim 13, or the medical preparation according to claim 16 and radiation.

22. A method of treating a tumor in a subject, comprising administering to the subject an effective amount of the antibody drug conjugate according to any one of claims 8-12 or the pharmaceutical composition according to claim 13 or the medical preparation according to claim 16 and an anti-proliferative agent.

23. A method for preparing a conjugate, comprising: linking the antibody or the function fragment thereof according to any one of claims 1-4 to a drug in the presence of a transglutaminase, wherein the linker is conjugated to an endogenous acceptor glutamine residue Q295 on the antibody.

24. The method according to claim 23, wherein the drug is selected from auristatin derivatives, maytansinoid derivatives, camptothecin analogues, DNA topoisomerase I inhibitors and derivatives thereof, preferably, the drug is selected from MMAE, MMAF, DM1, DM4, DXD and SN38 and derivatives thereof; said linker is selected from mc-Val-Cit-pABC, mc-Val-Cit, $NH_2$-$(PEG)_m$-Val-Cit and $NH_2$-$(PEG)m$-Val-Cit-pABC, wherein m is an integer from 1 to 8.

FIG. 1

FIG. 2

FIG. 3

FIG. 4

FIG. 5

FIG. 6

FIG. 7A

FIG. 7B

FIG. 8

FIG. 9

FIG. 10

FIG. 11

**Tumor Volume of HT1376**

vehicle
PADCEV 3mg/kg
SWY2001-Ab1-LND1002 3mg/kg

FIG. 12

## INTERNATIONAL SEARCH REPORT

| | International application No. |
|---|---|
| | **PCT/CN2022/114793** |

### A. CLASSIFICATION OF SUBJECT MATTER

C07K 16/28(2006.01)i; C12N 15/13(2006.01)i; A61K 39/395(2006.01)i; A61K 47/68(2017.01)i; A61K 45/06(2006.01)i; A61K 31/704(2006.01)i; A61P 35/00(2006.01)i

According to International Patent Classification (IPC) or to both national classification and IPC

### B. FIELDS SEARCHED

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, USTXT, EPTXT, CNKI, 万方数据库, WANFANG DATABASE, Pubmed, NCBI, EBI, 中国专利生物序列检索系统, Chinese Patent Biological Sequence Retrieval System: Nectin-4, 抗体, antibody, CDR, 可变区, 重链, 轻链, HV, LV, 缀合物, 偶联, 癌症, cancer, tumor, durg, ADC, SEQ ID NOs: 9-12, 石药集团巨石生物制药有限公司

### C. DOCUMENTS CONSIDERED TO BE RELEVANT

| Category* | Citation of document, with indication, where appropriate, of the relevant passages | Relevant to claim No. |
|---|---|---|
| PX | CN 113527486 A (MABWELL (SHANGHAI) BIOSCIENCE CO., LTD.) 22 October 2021 (2021-10-22) <br> claims 1-14, and embodiment 2 | 1-8, 11-22 |
| PY | CN 113527486 A (MABWELL (SHANGHAI) BIOSCIENCE CO., LTD.) 22 October 2021 (2021-10-22) <br> claims 1-14, and embodiment 2 | 9-10, 23-24 |
| Y | US 2018243434 A1 (INSERMINSTITUT NATIONAL DE LA SANTE ET DE LA RECHERCHE MEDICALE et al.) 30 August 2018 (2018-08-30) <br> embodiment 1 | 9-10, 23-24 |
| Y | CN 111093701 A (DEVELOPMENT CENTER FOR BIOTECHNOLOGY) 01 May 2020 (2020-05-01) <br> claims 1-12, and embodiment 2 | 10 |
| A | WO 2021069508 A1 (UNIVERSITE D'AIX-MARSEILLE et al.) 15 April 2021 (2021-04-15) <br> entire document | 1-24 |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **10 November 2022** | **25 November 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**EP 4 393 951 A1**

### INTERNATIONAL SEARCH REPORT

International application No.

**PCT/CN2022/114793**

**C. DOCUMENTS CONSIDERED TO BE RELEVANT**

| Category* | Citation of document, with indication, where appropriate, of the relevant passages | Relevant to claim No. |
|---|---|---|
| A | CN 112088167 A (YISSUM RESEARCH DEVELOPMENT COMPANY OF THE HEBREW UNIVERSITY OF JERUSALEM et al.) 15 December 2020 (2020-12-15)<br>entire document | 1-24 |
| A | WO 2019215728 A1 (YISSUM RESEARCH DEVELOPMENT COMPANY OF HEBREW UNIVERSITY OF JERUSALEM LTD. et al.) 14 November 2019 (2019-11-14)<br>entire document | 1-24 |
| A | US 2016151515 A1 (UNIVERSITE FRANCOIS RABELAIS) 02 June 2016 (2016-06-02)<br>entire document | 1-24 |
| A | CN 110156893 A (GENENTECH, INC.) 23 August 2019 (2019-08-23)<br>entire document | 1-24 |
| A | CHALLITA-EID, P. M. et al. "Enfortumab Vedotin Antibody–Drug Conjugate Targeting Nectin-4 Is a Highly Potent Therapeutic Agent in Multiple Preclinical Cancer Models"<br>*Cancer Research*, Vol. 76, No. 10, 15 May 2016 (2016-05-15),<br>pp. 3003-3013 | 1-24 |

Form PCT/ISA/210 (second sheet) (January 2015)

35

**INTERNATIONAL SEARCH REPORT**

International application No.

**PCT/CN2022/114793**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1]   The actually submitted sequence table is an XML file of the standard ST.26.

## INTERNATIONAL SEARCH REPORT

International application No.

**PCT/CN2022/114793**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **20-22**
   because they relate to subject matter not required to be searched by this Authority, namely:

   [1]  Claims 20-22 relate to a method for treating a tumor, and belong to disease treatment methods
        defined in PCT Rule 39.1(iv). Therefore, a search was conducted on the basis of a corresponding
        pharmaceutical use of an antibody drug conjugate and a pharmaceutical composition thereof.

2. ☐ Claims Nos.:
   because they relate to parts of the international application that do not comply with the prescribed requirements to such an
   extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

# EP 4 393 951 A1

<table>
<thead>
<tr><th colspan="3">INTERNATIONAL SEARCH REPORT<br>Information on patent family members</th><th>International application No.<br><br>PCT/CN2022/114793</th></tr>
</thead>
</table>

| Patent document<br>cited in search report | Publication date<br>(day/month/year) | Patent family member(s) | | Publication date<br>(day/month/year) |
|---|---|---|---|---|
| CN 113527486 A | 22 October 2021 | None | | |
| US 2018243434 A1 | 30 August 2018 | JP | 2018531913 A | 01 November 2018 |
| | | WO | 2017042210 A1 | 16 March 2017 |
| | | EP | 3347048 A1 | 18 July 2018 |
| | | ES | 2794557 T3 | 18 November 2020 |
| CN 111093701 A | 01 May 2020 | US | 2019106507 A1 | 11 April 2019 |
| | | TW | 201905000 A | 01 February 2019 |
| | | CA | 3067380 A1 | 20 December 2018 |
| | | WO | 2018232349 A1 | 20 December 2018 |
| | | JP | 2020524669 A | 20 August 2020 |
| | | EP | 3638306 A1 | 22 April 2020 |
| WO 2021069508 A1 | 15 April 2021 | AU | 2020364959 A1 | 07 April 2022 |
| | | IL | 291966 A | 01 June 2022 |
| | | KR | 20220079614 A | 13 June 2022 |
| | | CA | 3156451 A1 | 15 April 2021 |
| | | CN | 114514246 A | 17 May 2022 |
| | | EP | 4041765 A1 | 17 August 2022 |
| CN 112088167 A | 15 December 2020 | JP | 2021522801 A | 02 September 2021 |
| | | US | 2021130459 A1 | 06 May 2021 |
| | | RU | 2020133629 A | 10 June 2022 |
| | | WO | 2019215728 A1 | 14 November 2019 |
| | | CA | 3097679 A1 | 14 November 2019 |
| | | AU | 2019264965 A1 | 19 November 2020 |
| | | KR | 20210009308 A | 26 January 2021 |
| | | IL | 277858 A | 30 November 2020 |
| | | EP | 3790900 A1 | 17 March 2021 |
| WO 2019215728 A1 | 14 November 2019 | JP | 2021522801 A | 02 September 2021 |
| | | US | 2021130459 A1 | 06 May 2021 |
| | | RU | 2020133629 A | 10 June 2022 |
| | | CA | 3097679 A1 | 14 November 2019 |
| | | AU | 2019264965 A1 | 19 November 2020 |
| | | KR | 20210009308 A | 26 January 2021 |
| | | IL | 277858 A | 30 November 2020 |
| | | EP | 3790900 A1 | 17 March 2021 |
| | | CN | 112088167 A | 15 December 2020 |
| US 2016151515 A1 | 02 June 2016 | CA | 2917544 A1 | 15 January 2015 |
| | | EP | 3019202 A1 | 18 May 2016 |
| | | WO | 2015004400 A1 | 15 January 2015 |
| | | JP | 2016531094 A | 06 October 2016 |
| | | US | 2019365914 A1 | 05 December 2019 |
| | | EP | 3521314 A1 | 07 August 2019 |
| | | ES | 2874857 T3 | 05 November 2021 |
| | | FR | 3008408 A1 | 16 January 2015 |
| | | CN | 105592859 A | 18 May 2016 |
| | | EP | 3825304 A1 | 26 May 2021 |
| CN 110156893 A | 23 August 2019 | MX | 2020001752 A | 15 January 2021 |
| | | JP | 2022137073 A | 21 September 2022 |
| | | TW | 202118786 A | 16 May 2021 |
| | | AU | 2014364805 A1 | 16 June 2016 |

Form PCT/ISA/210 (patent family annex) (January 2015)

**INTERNATIONAL SEARCH REPORT**
Information on patent family members

| International application No. |
| --- |
| **PCT/CN2022/114793** |

| Patent document cited in search report | Publication date (day/month/year) | Patent family member(s) | | | Publication date (day/month/year) |
| --- | --- | --- | --- | --- | --- |
| | | CN | 106029696 | A | 12 October 2016 |
| | | JP | 2021006042 | A | 21 January 2021 |
| | | CA | 2932288 | A1 | 25 June 2015 |
| | | SI | 3192812 | T1 | 30 October 2020 |
| | | PE | 20210648 | A1 | 26 March 2021 |
| | | BR | 112016014022 | A2 | 01 October 2019 |
| | | LT | PA2022519 | I1 | 10 October 2022 |
| | | CL | 2016001556 | A1 | 03 February 2017 |
| | | RS | 60443 | B1 | 31 July 2020 |
| | | HR | P20201143 | T1 | 30 October 2020 |
| | | WO | 2015095392 | A1 | 25 June 2015 |
| | | MX | 2016007958 | A | 03 August 2016 |
| | | PE | 20210107 | A1 | 19 January 2021 |
| | | PT | 3192812 | T | 28 August 2020 |
| | | LT | 3192812 | T | 10 August 2020 |
| | | NZ | 721309 | A | 29 November 2019 |
| | | PL | 3083689 | T3 | 19 October 2020 |
| | | LT | 3083689 | T | 10 August 2020 |
| | | PL | 3192812 | T3 | 19 October 2020 |
| | | TW | 201902932 | A | 16 January 2019 |
| | | US | 2020299408 | A1 | 24 September 2020 |
| | | JP | 2019129831 | A | 08 August 2019 |
| | | DK | 3192812 | T3 | 13 July 2020 |
| | | IL | 263466 | A | 31 January 2019 |
| | | HU | E050156 | T2 | 30 November 2020 |
| | | AU | 2020200101 | A1 | 30 January 2020 |
| | | US | 2017204194 | A1 | 20 July 2017 |
| | | US | 2019016823 | A1 | 17 January 2019 |
| | | US | 2020299409 | A1 | 24 September 2020 |
| | | KR | 20210028735 | A | 12 March 2021 |
| | | CA | 3131724 | A1 | 25 June 2015 |
| | | AU | 2022203605 | A1 | 16 June 2022 |
| | | HU | E050159 | T2 | 30 November 2020 |
| | | EA | 201691266 | A1 | 31 May 2017 |
| | | JP | 2022137074 | A | 21 September 2022 |
| | | CN | 112390883 | A | 23 February 2021 |
| | | ES | 2813074 | T3 | 22 March 2021 |
| | | TW | 201527323 | A | 16 July 2015 |
| | | UA | 120753 | C2 | 10 February 2020 |
| | | CA | 3055708 | A1 | 25 June 2015 |
| | | KR | 20160098464 | A | 18 August 2016 |
| | | SG | 10201800250 X | A | 27 February 2018 |
| | | NZ | 758346 | A | 29 October 2021 |
| | | SI | 3083689 | T1 | 30 October 2020 |
| | | EP | 3192812 | A1 | 19 July 2017 |
| | | EP | 3736292 | A1 | 11 November 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022051591 A2

**Non-patent literature cited in the description**

- **LAURENT DUCRY.** Antibody Drug Conjugates. Science Press, 36-37
- **CHEN JIANJUN et al.** *Journal of Cellular and Molecular Immunology,* 1997, (3
- **HARLOW ; LANE.** Antibodies: A Laboratory Manual. Cold Spring Harbor Laboratory, 1988, 726
- *Nature,* 1975, vol. 256, 495-497
- Chinese Pharmacopoeia. 2020